(19) Europäisches Patentamt
European Patent Office
Office européen des brevets

(11) EP 0 874 818 B1

(12) EUROPEAN PATENT SPECIFICATION

(45) Date of publication and mention of the grant of the patent:
**21.05.2003 Bulletin 2003/21**

(21) Application number: **96940819.4**

(22) Date of filing: **14.11.1996**

(51) Int Cl.[7]: **C07D 207/08**, C07D 207/12, C07D 209/94, C07D 211/54, C07D 401/02, A61K 31/40, A61K 31/445

(86) International application number:
**PCT/US96/18569**

(87) International publication number:
**WO 97/019059 (29.05.1997 Gazette 1997/23)**

# (54) NOVEL SUBSTITUTED ARYL COMPOUNDS USEFUL AS MODULATORS OF ACETYLCHOLINE RECEPTORS

NEUE SUBSTITUIERTE ARYL-VERBINDUNGEN ALS ACETYLCHOLINE RECEPTORS MODULATOREN

NOUVEAUX COMPOSES D'ARYLE SUBSTITUES UTILES COMME MODULATEURS DES RECEPTEURS DE L'ACETYLCHOLINE

(84) Designated Contracting States:
**AT BE CH DE DK ES FI FR GB GR IE IT LI LU MC NL PT SE**

(30) Priority: **17.11.1995 US 559821**

(43) Date of publication of application:
**04.11.1998 Bulletin 1998/45**

(73) Proprietor: **MERCK & CO. INC.**
**Rahway New Jersey 07065-0900 (US)**

(72) Inventors:
- **VERNIER, Jean, Michel**
  **San Diego, CA 92128 (US)**
- **MCDONALD, Ian, A.**
  **San Diego, CA 92130 (US)**

(74) Representative: **Horgan, James Michael Frederic**
**Merck & Co., Inc.,**
**Terlings Park,**
**Eastwick Road**
**Harlow, Essex CM20 2QR (GB)**

(56) References cited:
**WO-A-94/07489**
**WO-A-96/11931**
**US-A- 4 721 783**
**US-A- 5 472 958**

- **BIOORG. MED. CHEM. LETT. (1996), 6(19), 2283-2288 CODEN: BMCLE8;ISSN: 0960-894X, 1996, XP000618280 ELLIOTT, RICHARD L. ET AL: "2-(Aryloxymethyl) azacyclic analogs as novel nicotinic acetylcholine receptor (nAChR) ligands"**
- **J. ENVIRON. SCI. HEALTH, PART B (1983), B18(4-5), 515-27 CODEN: JPFCD2;ISSN: 0360-1234, 1983, XP000618288 KASZUBSKA, J. ET AL: "Fungicidal activity of 2-[2-(arylthio)ethyl]pyrrolidine derivatives"**
- **POL. J. CHEM. (1981), 55(7-8), 1681-4 CODEN: PJCHDQ, 1981, XP000618279 BAL, STANISLAW ET AL: "Synthesis of 1-acyl-2-[2-(S-arylthio)ethyl]pyrrolidines "**
- **POL. J. CHEM. (1982), 56(10-12), 1543-7 CODEN: PJCHDQ, 1982, XP000618493 KASZUBSKA, JANINA ET AL: "Synthesis of derivatives of 2-[2-(arylthio)ethyl]pyrrolidine"**

EP 0 874 818 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

The present invention relates to compounds which potentiate neurotransmission by promoting the release of neurotransmitters such as acetylcholine, dopamine and norepinephrine. More particularly, the present invention relates to compounds that are capable of modulating acetylcholine receptors. Invention compounds are useful, for example, for treatment of dysfunction of the central and autonomic nervous systems (e.g. dementia, cognitive disorders, neurodegenerative disorders, extrapyramidal disorders, convulsive disorders, cardiovascular disorders, endocrine disorders, eating disorders, affective disorders and drug abuse). In addition, the present invention relates to pharmaceutical compositions containing these compounds, as well as various uses therefor.

## BACKGROUND OF THE INVENTION

By modulating neurotransmitter release (including dopamine, norepinephrine, acetylcholine and serotonin) from different brain regions, acetylcholine receptors are involved in the modulation of neuroendocrine function, respiration, mood, motor control and function, focus and attention, concentration, memory and cognition, and the mechanisms of substance abuse. Ligands for acetylcholine receptors have been demonstrated to have effects on attention, cognition, appetite, substance abuse, memory, extrapyramidal function, cardiovascular function, pain and gastrointestinal motility and function. The distribution of acetylcholine receptors that bind nicotine, i.e., nicotinic acetylcholine receptors, is widespread in the brain, including the basal ganglia, limbic system, cerebral cortex and mid- and hindbrain nuclei. In the periphery, the distribution includes muscle, autonomic ganglia, the gastrointestinal tract and the cardiovascular system.

Acetylcholine receptors have been shown to be decreased, *inter alia,* in the brains of patients suffering from Alzheimer's disease or Parkinson's disease, diseases associated with dementia, motor dysfunction and cognitive impairment. Such correlations between acetylcholine receptors and nervous system disorders suggest that compounds that modulate acetylcholine receptors will have beneficial therapeutic effects for many human nervous system disorders. Thus, there is a continuing need for compounds which have the ability to modulate the activity of acetylcholine receptors. In response to such need, the present invention provides a new family of compounds which modulate acetylcholine receptors.

## BRIEF DESCRIPTION OF THE INVENTION

In accordance with the present invention, we have discovered a novel class of substituted aryl compounds (containing an ether, ester, amide, ketone or thioether functionality) that promote the release of ligands involved in neurotransmission. More particularly, compounds of the present invention are capable of modulating acetylcholine receptors.

The compounds of the present invention are capable of displacing one or more acetylcholine receptor ligands, e.g., $^{3}$H-nicotine, from mammalian neuronal membrane binding sites. In addition, invention compounds display activity in cell lines which express recombinant acetylcholine receptors. It can readily be seen, therefore, that invention compounds may act as agonists, partial agonists, antagonists or allosteric modulators of acetylcholine receptors. Therapeutic indications for compounds with activity at acetylcholine receptors include diseases of the central nervous system such as Alzheimer's disease and other diseases involving memory loss and/or dementia (including AIDS dementia); cognitive dysfunction (including disorders of attention, focus and concentration), disorders of extrapyramidal motor function such as Parkinson's disease, progressive supramuscular palsy, Huntington's disease, Gilles de la Tourette syndrome and tardive dyskinesia; mood and emotional disorders such as depression, anxiety and psychosis; substance abuse including withdrawal symptoms and substitution therapy; neurocrine disorders and dysregulation of food intake, including bulimia and anorexia; disorders or nociception and control of pain; autonomic disorders including dysfunction of gastrointestinal motility and function such as inflammatory bowel disease, irritable bowel syndrome, diarrhea, constipation, gastric acid secretion and ulcers; pheochromocytoma, cardiovascular dysfunction including hypertension and cardiac arrhythmias, as well as co-medication uses in surgical applications.

## BRIEF DESCRIPTION OF THE FIGURES

Figure 1 illustrates the effect of saline injection on ACh release in the hippocampus. Saline (1.0 ml/kg) was injected subcutaneously at time = 0 and acetylcholine levels measured as described in Example 7 (n=4 animals).

Figure 2 illustrates acetylcholine release in the hippocampus induced by nicotine. Nicotine (0.4 mg/kg) was injected

at time 0, and acetylcholine levels measured as described in Example 7. Statistical significance was determined using students t-test versus saline control animals (*P<0.05, n=4 animals).

Figure 3 illustrates acetylcholine release in the hippocampus induced by lobeline. Lobeline (5.0 mg/kg) was injected at time 0, and acetylcholine levels measured as described in Example 7. Statistical significance was determined using students t-test versus saline control animals (*P<0.05, n=3 animals).

Figure 4 illustrates acetylcholine release in the hippocampus induced by the compound of Formula Z (as defined hereinafter) wherein A = 4-hydroxyphenyl, B is not present, D = -S-, E = $-CH_2CH_2-$, and G = 1-methylpyrrolidino. This compound (40 mg/kg) was injected at time 0, and acetylcholine levels measured as described in Example 7. Statistical significance was determined using students t-test versus saline control animals (*P<0.05, n=3 animals).

Figure 5 illustrates acetylcholine release in the hippocampus induced by the above-described compound of Formula Z (closed squares), mecamylamine and the D1 dopamine antagonist, SCH22390 (RBI, Inc., Nadick, MA).

DETAILED DESCRIPTION OF THE INVENTION

In accordance with the present invention, there are provided compounds having Formula Z, as follows:

$$A\text{-}B\text{-}D\text{-}E\text{-}G \qquad (Z)$$

or enantiomers, diastereomeric isomers or mixtures of any two or more thereof, or pharmaceutically acceptable salts thereof,
wherein:

A is

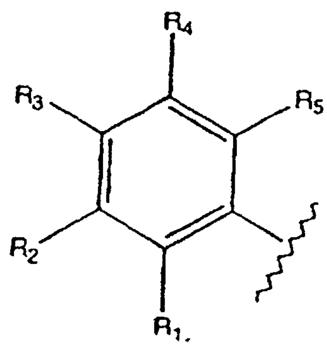

wherein:

each of $R_1$, $R_2$, $R_3$, $R_4$ and $R_5$ are independently selected from hydrogen, halogen, $C_{1-4}$ alkyl or $-OR_A$, wherein $R_A$ is selected from H, $C_{1-4}$ alkyl or $C_{6-14}$ aryl, and wherein at least one of $R_1$, $R_2$, $R_3$, $R_4$ or $R_5$ is not hydrogen;

B is optionally present; with the proviso that when B is absent and D is -O-, at least one of $R_1$, $R_2$, $R_3$, $R_4$ or $R_5$ is not hydrogen, and when B is present, B is selected from $C_{1-4}$ alkylene, $C_{3-8}$ cycloalkylene, $C_{2-4}$ alkenylene, or $C_{2-4}$ alkynylene;
D is selected from -O-, -C(O)-, -C(O)-O- or -S-;
E is selected from $C_{1-4}$ alkylene, $C_{2-4}$ alkenylene or $C_{2-4}$ alkynylene, with the proviso that when any one of $R_1$, $R_2$, $R_3$, $R_4$ or $R_5$ is halogen, and D is -O-, then E is not methylene;
G is a dialkylamino group having the structure:

$$-N(R^E)\ (R^F),$$

wherein:

$R^E$ is hydrogen or a $C_{1-4}$ alkyl, and
$R^F$ is hydrogen or $C_{1-4}$ alkyl,
with the proviso that when G is dialkylamino, B is absent, D is -O-, and E is $C_{1-3}$ alkylene, then at least one of $R^1$ and $R^5$ is not $C_{1-4}$ alkyl, or

G is a nitrogen-containing cyclic moiety having the structure:

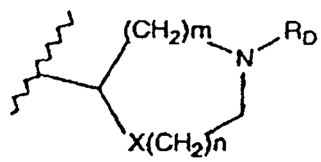

wherein:

m is 0-2,
n is 0-3,
X is optionally present, and when present is selected from -O-, $-CH_2O-$, -S-, $-CH_2S-$, -S(O)-, $-CH_2S(O)-$, $-S(O)_2-$, $-CH_2S(O)_2-$ or $-CH_2NH-$, wherein n is not 0 when X is not present, such that G is an aziridino, azetidino, tetrahydrooxazolo, tetrahydrothiazolo, pyrrolidino, piperidino, morpholino, thiomorpholino, piperazino, 7-azabicyclo[2.2.1]heptano, 8-azabicyclo[3.2.1]octano, 1-azabicyclo[2.2.2]octano or 9-azabicyclo[4.2.1]nonane moiety and
$R_D$ is selected from hydrogen, $C_{1-4}$ alkyl or $C_{3-4}$ cycloalkyl, or $R_D$ is absent when the nitrogen atom to which it is attached participates in the formation of a double bond, with the proviso that when D is -S-, then $R_D$ is not hydrogen or methyl,
with the proviso that:

when B is not present, D is -O- E is a $C_{1-3}$ alkylene, and G is a nitrogen-containing cyclic moiety wherein X is not present, then m and n combined ≠ 1; and
when B is not present, D is -S-, E is $-CH_2CH_2-$, G is a nitrogen-containing cyclic moiety wherein X is not present, m is zero, n is one and $R_D$ is $CH_3$, and each of $R_1$, $R_2$, $R_4$ and $R_5$ is H, then $R_3$ is not H, Cl or tert-butyl.

As employed herein, "$C_{1-4}$ alkyl" refers to straight or branched chain alkyl radicals;

"$C_{1-4}$ alkylene" refers to straight or branched chain alkylene radicals (i.e., divalent alkyl moieties, e.g., methylene);
"$C_{3-8}$ cycloalkylene" refers to cyclic ring-containing divalent radicals (e.g. cyclohexylene);
"$C_{2-4}$ alkenylene" refers to straight or branched chain alkenylene radicals (i.e., divalent alkenyl moieties, e.g., ethylidene) having at least one carbon-carbon double bond;
"$C_{2-4}$ alkynylene" refers to straight or branched chain alkynylene radicals (i.e., divalent alkynyl moieties, e.g., ethynylidene) having at least one carbon-carbon triple bond; and
"halogen" refers to fluoride, chloride, bromide or iodide radicals.

Presently preferred moieties for B are alkylene chains containing 1 to 3 carbon atoms in the backbone thereof.
It is presently especially preferred that D is selected from -S- or -C(O)O-.
Presently preferred moieties for E are alkylene of 1 to 3 carbon atoms.
Presently preferred compounds include those wherein G is an azetidino moiety, pyrrolidino moiety, 1-methylpyrrolidino moiety, 7-azabicyclo[2.2.1]heptane, 8-azabicyclo[3.2.1]octane, 1-azabicyclo[2.2.2]octane and 9-azabicyclo[4.2.1]nonane.
Preferred compounds of the present invention include those wherein D is -S-; A is 4-hydroxypeheny, 2-fluoro-4-hydroxyphenyl, 2-chloro-4-hydroxyphenyl or 3-fluoro-4-methoxyphenyl; B is absent; E is $C_{1-4}$ alkylene; and, G forms an azetidine, tetrahydrooxazole, tetrahydrothiazole, pyrrolidine, piperidine, morpholine, thiomorpholine or piperazine ring. Particularly preferred compounds of the present invention include those wherein D is -S-; A is 4-hydroxyphenyl,

2-chloro-4-hydroxyphenyl or 3-fluoro-4-methoxyphenyl; B is absent; E is ethylene; and G is pyrrolidino or 1-methylpyrrolidino.

Additional preferred compounds of the present invention include those wherein D is -C(O)O-; A is 4-hydroxyphenyl; B is ethylene or propylene; E is methylene; and G is pyrrolidino or 1-methylpyrrolidino.

Additional preferred compounds of the invention include those wherein D is -S-; B is not present; E is ethylene; G is pyrrolidino; and A is 2-methyl-4-hydroxyphenyl.

Additional preferred compounds of the invention include those wherein D is -O-; B is methylene or not present; E is methylene; G is pyrrolidino; and A is 4-hydroxyphenyl.

Additional preferred compounds of the invention include those wherein D is -S-; B is methylene or not present; E is methylene; and A is an R-substituted phenyl (wherein R is defined the same as any one of $R_1$, $R_2$, $R_3$, $R_4$ or $R_5$, e.g., 4-hydroxyphenyl, 4-methoxyphenyl).

Additional preferred compounds of the invention include those wherein D is -S-; B is not present; E is methylene; A is hydroxyphenyl; and G is 7-azabicyclo[2.2.1]heptane, 8-azabicyclo[3.2.1]octane, 1-azabicyclo[2.2.2]-octane, or 9-azabicyclo[4.2.1]nonane.

Additional preferred compounds of the invention include those wherein D is -S-; B is not present; E is $-(CH_2)_n-$, wherein n - 1-4, e.g., methylene, ethylene, propylene, butylene; A is 4-hydroxyphenyl; and G is dialkylamino (e.g., dimethylamino), pyrrolidino, piperidino, 7-azabicyclo[2.2.1]heptano, 8-azabicyclo[3.2.1]octano, 1-azabicyclo[2.2.2]octano or 9-azabicyclo[4.2.1]nonane.

In the following reaction Schemes, each of A, B, D, E and G are as defined above. When any one or more of the R-group substituents of A (i.e., $R_1$, $R_2$, $R_3$, $R_4$ or $R_5$) is -OH it will be readily apparent to those of , skill in the art that this functional group may require the use of "protecting groups" (e.g., t-butyldimethylsilyl (t-BDMS), benzyl ($B_n$) or tetrahydrophenyl (THP)) during the coupling reaction to "block" the reactivity of the R group. Furthermore, when G = pyrrolidine (i.e., $R_D$=H), an additional protecting step may be required. For such purpose, BOC, CBZ, and the like can be employed. Hence, subsequent deprotection will be required prior to analysis.

Alternative methods for the preparation of compounds having the general Formula Z, as follows:

$$A\text{-}B\text{-}D\text{-}E\text{-}G \qquad (Z)$$

as described herein above, wherein D is present and represents an ester linking moiety (i.e., -C(O)O-), are shown in Reaction Schemes I, II and III. In Reaction Scheme I, compounds of Formula I, wherein B is absent or selected from methylene or ethylene, are commercially available and are well known to those of skill in the art. Those compounds not currently available may readily be prepared from starting materials well-known to those of skill in the art.

## Reaction Scheme I

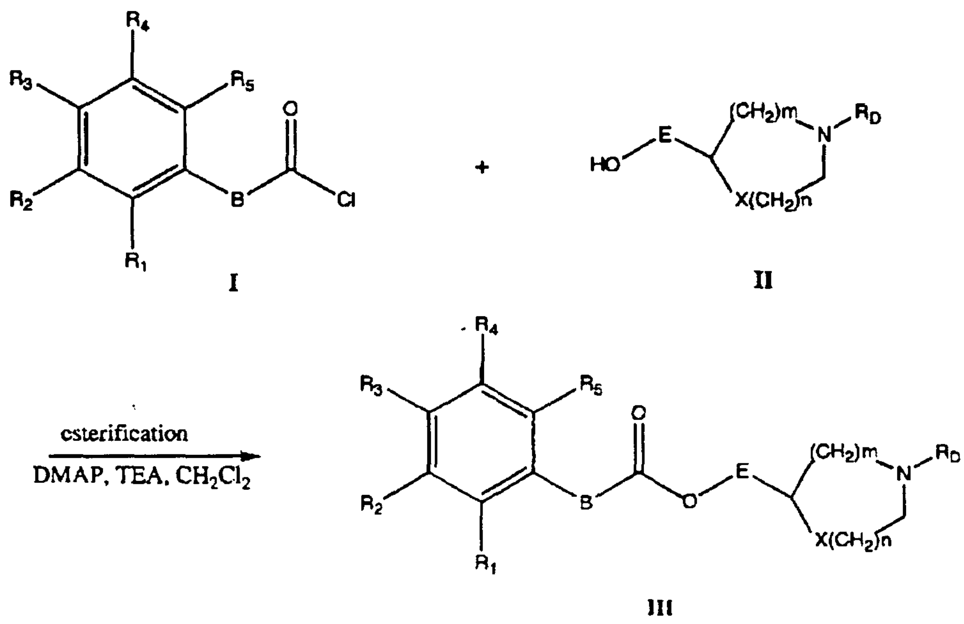


I II III

In Reaction Scheme I, the aryl acid chlorides of Formula I are effectively contacted with the primary alcohol compounds of Formula II, optionally bearing E, and dimethylaminopyridine (DMAP) under anhydrous conditions in an aprotic solvent, such as, for example, methylene chloride ($CH_2Cl_2$), tetrahydrofuran (THF), ether, diethyl ether, benzene, toluene, and the like. Compounds of Formula II are commercially available, or can be prepared from readily available starting materials, employing techniques well-known to those of skill in the art. See, for example, Kreug and Reinecke, *J. Org. Chem.* **32:**225 (1967); Eremeev et *al., Chem. Heterocycl. Compd.* (English Translation) **22:**1039-1044 (1986); Morie *et al., J. Chem. Soc. Perkin Trans.* **1:**2565-2570 (1994); Habermehl and Ecsy, *Heterocycles* **7:**1027-1032 (1977); and Brown *et al., J. Chem. Soc. Perkin Trans.* **1:**2577-2580 (1985)). Similarly, hydroxy derivatives of dialkylamines or azabicycloalkanes can be used instead of compounds of Formula II. The reaction mixtures are stirred for 1 to 16 hr, with 4 hours preferred, at reaction temperatures within the range of -78°C up to ambient, with ambient temperatures presently preferred. The resulting esters (Formula III) are typically purified, e.g., by chromatography over silica, and the final product analyzed by NMR.

Alternatively, compounds of Formula III may be prepared by the trans-esterification reaction described in Reaction Scheme II.

## Reaction Scheme II

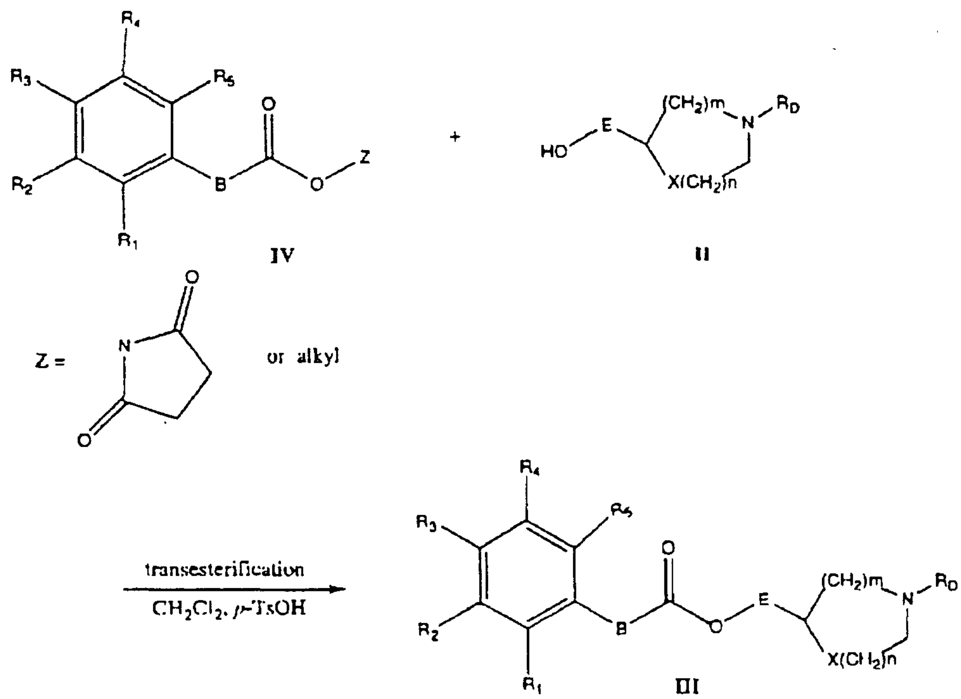


IV

II

III

In Reaction Scheme II, aryl esters of Formula IV, optionally containing B, are effectively contacted with compounds of Formula II, optionally bearing E, in the presence of an aprotic solvent (e.g., methylene chloride or benzene) and a catalytic amount of p-toluenesulfonic acid (p-TsOH), to afford the ester compounds of Formula III. Similarly, hydroxy derivatives of dialkylamines or azabicycloalkanes can be used instead of compounds of Formula II. The reaction mixture is refluxed (i.e., boiled) in the range of 8 to 16 hours, with 12 hours presently preferred, and the resulting ester is purified and analyzed by NMR.

Further, compounds of Formula III may be prepared from aryl carboxylic acid derivatives according to Reaction Scheme III.

## Reaction Scheme III

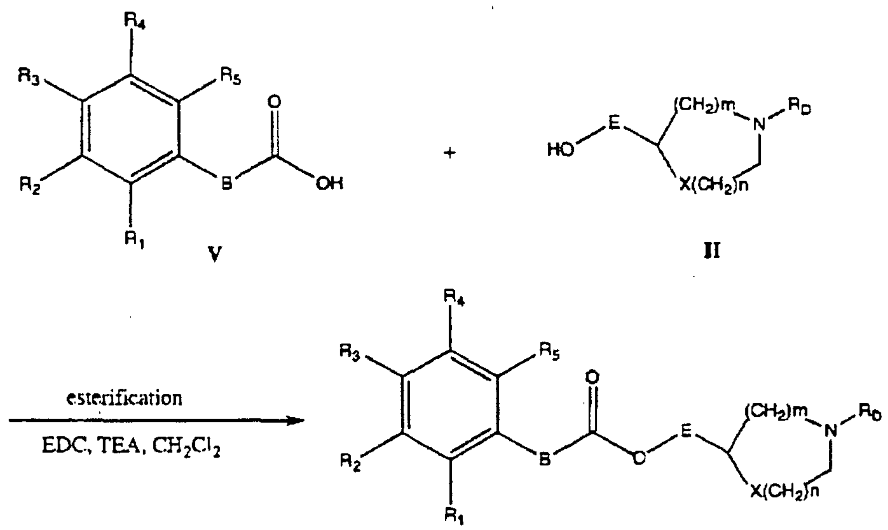


V

II

III

Carboxylic acid derivatives V employed in Reaction Scheme III are commercially available or may readily be prepared from well-known starting materials. Compounds of Formula V are coupled with compounds of Formula II in the presence of triethylamine (TEA), 1-(3-dimethylaminopropyl)-3-ethylcarbodiimide (EDC) in an aprotic solvent such as methylene chloride ($CH_2Cl_2$) or chloroform and the like. Similarly, hydroxy derivatives of dialkylamines or azabicycloalkanes can be used instead of compounds of Formula II. The reaction mixtures are stirred for 8 to 16 hr, with 12 hr preferred, at reaction temperatures within the range of -78°C to ambient, with ambient temperatures presently preferred, to afford compounds III. The resulting esters are typically purified by chromtography over silca and the final product analyzed by NMR.

Reaction Scheme IV illustrates the preparation of compounds having the general Formula Z, as follows:

$$A\text{-}B\text{-}D\text{-}E\text{-}G \qquad (Z)$$

as described hereinabove, wherein D is a thioether.

Reaction Scheme IV

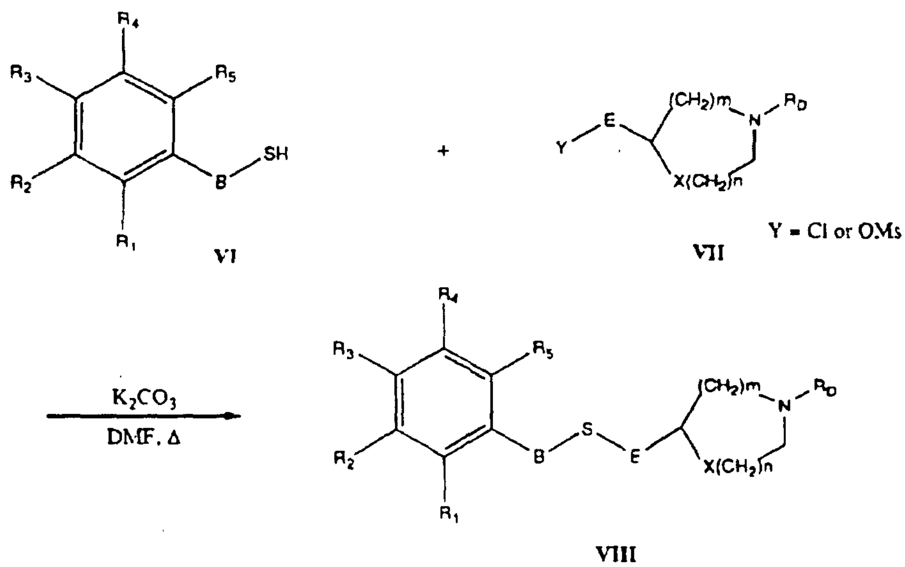

VI VII VIII

In reaction Scheme IV the sulfhydryl derivatives of A, and A optionally bearing B, (compounds VI) are commercially available (e.g., thiophenyl, 4-hydroxythiophenyl and 2-phenylethanethiol, Aldrich Chemical Co.) or may readily be prepared by those of skill in the art by selecting the appropriate A moiety.

In Reaction Scheme IV, the sulfur compounds (compounds VI) are effectively contacted with the chloride derivatives of G, optionally bearing E. compounds VII are commercially available or may be prepared from starting materials well-known to those of skill in the art (see e.g., Wrobel and Hejchman, *Synthesis* **5**:452 (1987) or Gautier *et al., Am. Pharm. Fr.* **30**:715 (1972)) or, alternatively, the mesylate derivative of compounds II may be used (as prepared according to Fürst and Koller (1947) Helv. Chim. Acta 30, 1454). Similarly, chloro or mesylate derivatives of dialkylamines or azabicycloalkanes can be used instead of compounds of Formula VII. This coupling reaction is promoted by suitable base, such as, for example potassium hydroxide, sodium ethoxide, potassium carbonate and 1,8-diazatricyclo[5.4.0]undec-7-ene (DBU). Presently preferred base for use in the practice of the present invention is potassium carbonate. The above-desired reaction is typically carried out in a solvent such as methanol, tetrahydrofuran (THF) and dimethylformamide (DMF). Presently preferred solvent for use in the practice of the present invention is dimethylformamide (DMF).

Typically the coupling reaction can be carried out over a wide range of temperatures. Temperatures in the range of about 80°C are presently preferred. Reaction times required to effect the desired coupling reaction can vary widely, typically falling in the range of 10 minutes up to about 24 hours. Preferred reaction times fall in the range of about 30 minutes to one hour. The resulting sulfur compound is purified and analyzed by NMR.

Alternative methods for the preparation of compounds wherein D is a ketone (i.e., -C(O)-) are shown in Reaction Schemes VII and VIII.

## Reaction Scheme VII

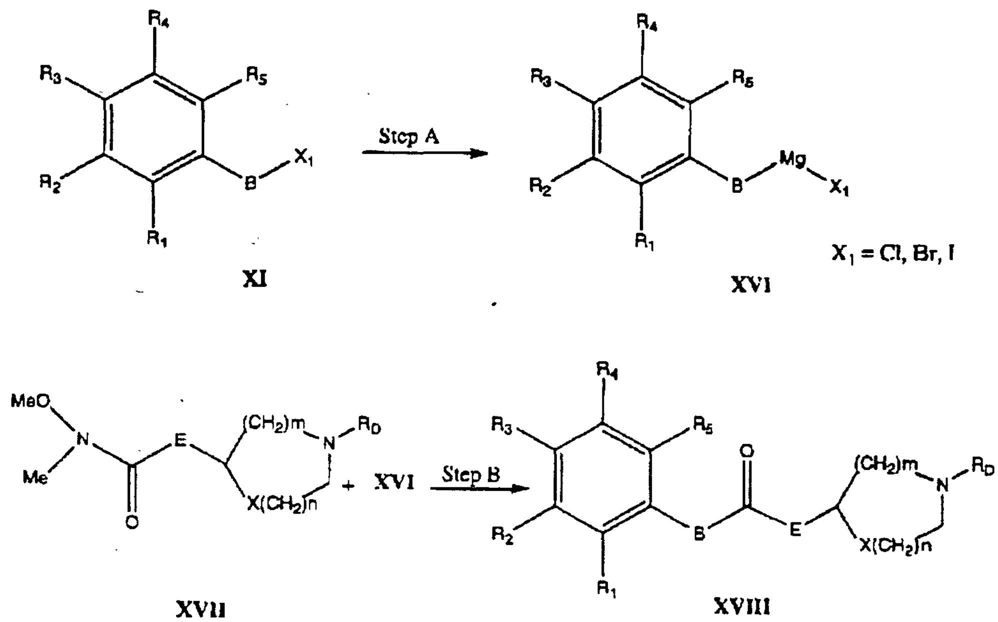


XI XVI XVII XVIII

As illustrated in Step A of Reaction Scheme VII, halogenated derivatives of A, optionally bearing B (compounds XI) are commercially available (e.g., phenylchloride, Aldrich Chemical Co.), or may readily be prepared by those of skill in the art, are reacted with magnesium in an aprotic solvent such as ether, tetrahydrofuran, benzene and the like, forming the corresponding Grignard reagent (compounds XVI). Presently preferred solvent for use in the practice of the present invention is tetrahydrofuran. Typically this reaction may be carried out over a wide range of temperatures. Temperatures in the range of about 65°C are presently preferred. Reaction times required to effect the desired reaction can vary widely, typically falling in the range of one hour to about 12 hours. Preferred reaction times fall in the range of 2 hours.

In step B of Reaction Scheme VI, compound XVII (see Scheme VII) can be contacted with Grignard reagent XVI to afford ketones XVIII (see S. Nahm and S. Weinreb, *Tet lett* **22:**3815 (1981)). Typically this reaction may be carried out in an aprotic solvent such as tetrahydrofuran, ether and the like. Presently preferred solvent for use in the practice of the present invention is tetrahydrofuran. Typically this reaction may be carried out over a wide range of temperatures. Temperatures in the range of 0°C are presently preferred. Reaction times required to effect the desired coupling can vary widely, typically in the range of one hour. The resulting products are purified and analyzed by NMR.

A method for the preparation of compounds of Formula XVII is depicted in Scheme VIII.

## Reaction Scheme VIII

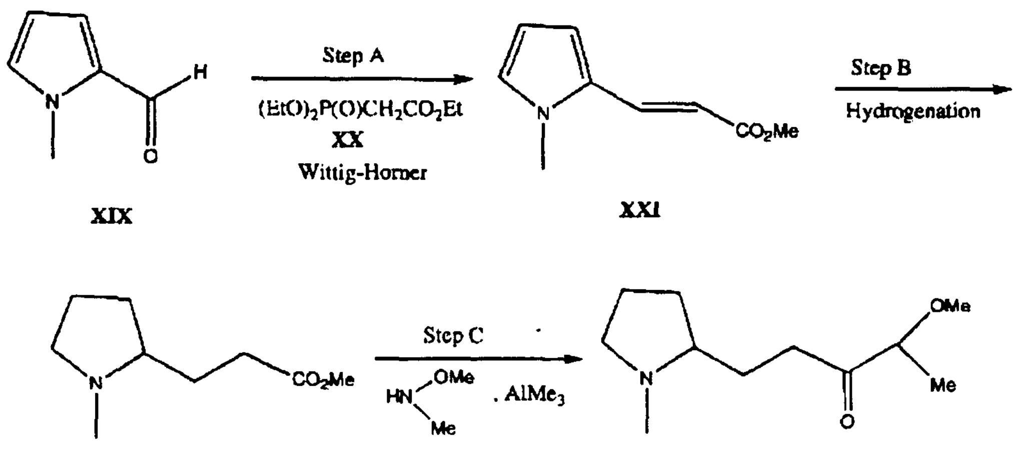


XIX XXI XXII XVII

In Step A of reaction Scheme VIII, aldehyde XIX is contacted with triethyl phosphonoacetate XX, via a Wittig-Horner reaction well known to those of skill in the art in order to obtain the unsaturated ester (compound XXI).

In step B of reaction Scheme VIII the resulting unsaturated ester (compound XXI) may be reduced to the corresponding saturated ester (compound XVII) using procedures well known to those of skill in the art, such as catalytic hydrogenation using a pressure of hydrogen, a catalyst such as $PtO_2$ and acetic acid as solvent.

In Step C of the reaction Scheme VIII the saturated ester (compound XXII) is contacted with N-methoxy-N-methylamine in the presence of trimethylaluminium in an aprotic solvent such as benzene in order to form the corresponding amide (compound XVII) (Levin et *al.*, *Synt. Com.* **12:**989 (1982)).

In addition to the above-described synthetic procedures, those of skill in the art have access to numerous other synthetic procedures which can be employed for the preparation of invention compounds. Indeed, the literature is replete with methodologies that can be used for the preparation of starting and/or intermediate compounds which are useful for the preparation of invention compounds (e.g., compounds having Formula **II, VI, XI, XIV, XVII, XXII,** and the like). Such starting and/or intermediate compounds can then be modified, for example, as described herein, to introduce the necessary substituents to satisfy the requirements of Formula I.

In accordance with another embodiment of the present invention, there are provided pharmaceutical compositions comprising substituted aryl compounds as described above, in combination with pharmaceutically acceptable carriers. Optionally, invention compounds can be converted into non-toxic acid addition salts, depending on the substituents thereon. Thus, the above-described compounds (optionally in combination with pharmaceutically acceptable carriers) can be used in the manufacture of a medicament for modulating the activity of acetylcholine receptors.

Pharmaceutically acceptable carriers contemplated for use in the practice of the present invention include carriers suitable for oral, intravenous, subcutaneous, transcutaneous, intramuscular, intracutaneous, inhalation, and the like administration. Administration in the form of creams, lotions, tablets, dispersible powders, granules, syrups, elixirs, sterile aqueous or non-aqueous solutions, suspensions or emulsions, patches, and the like, is contemplated. Also contemplated is single dose administration, sustained release administration (e.g., employing time release formulations, metered delivery, repetitive administration and continuous delivery), administration in combination with other active ingredients, and the like.

For the preparation of oral liquids, suitable carriers include emulsions, solutions, suspensions, syrups, and the like, optionally containing additives such as wetting agents, emulsifying and suspending agents, sweetening and flavoring and perfuming agents.

For the preparation of fluids for parenteral administration, suitable carriers include sterile aqueous or non-aqueous solutions, suspensions, or emulsions. Examples of non-aqueous solvents or vehicles are propylene glycol, polyethylene glycol, vegetable oils, such as olive oil and corn oil, gelatin, and injectable organic esters such as ethyl oleate. Such dosage forms may also contain adjuvants such as preserving, wetting, emulsifying, and dispersing agents. They may be sterilized, for example, by filtration through a bacteria-retaining filter, by incorporating sterilizing agents into the compositions, by irradiating the compositions, or by heating the compositions. They can also be manufactured in the form of sterile water, or some other sterile injectable medium immediately before use.

Invention compounds can optionally be converted into non-toxic acid addition salts. Such salts are generally prepared by reacting the compounds of this invention with a suitable organic or inorganic acid. Representative salts include the hydrochloride, hydrobromide, sulfate, bisulfate, methanesulfonate, acetate, oxalate, valerate, oleate, laurate, borate, benzoate, lactate, phosphate, tosylate, citrate, maleate, fumarate, succinate, tartrate, napsylate, and the like. Such salts can readily be prepared employing methods well known in the art.

. In accordance with yet another embodiment of the present invention, there are provided methods of modulating the activity of acetylcholine receptors, said method comprising:

contacting cell-associated acetylcholine receptors with a concentration of a pyridine compound as described above sufficient to modulate the activity of said acetylcholine receptors.

As employed herein, the phrase "modulating the activity of acetylcholine receptors" refers to a variety of therapeutic applications, such as the treatment of Alzheimer's disease and other disorders involving memory loss and/or dementia (including AIDS dementia); cognitive dysfunction (including disorders of attention, focus and concentration), disorders of extrapyramidal motor function such as Parkinson's disease, progressive supramuscular palsy, Huntington's disease, Gilles de la Tourette syndrome and tardive dyskinesia; mood and emotional disorders such as depression, panic, anxiety and psychosis; substance abuse including withdrawal syndromes and substitution therapy; neuroendocrine disorders and dysregulation of food intake, including bulemia and anorexia; disorders of nociception and control of pain; autonomic disorders including dysfunction of gastrointestinal motility and function such as inflammatory bowel disease, irritable bowel syndrome, diarrhea, constipation, gastric acid secretion and ulcers; pheochromocytoma; and cardiovascular dysfunction including hypertension and cardiac arrhythmias, comedication in surgical procedures.

The compounds of the present invention are especially useful for the treatment of Alzheimer's disease as well as other types of dementia (including dementia associated with AIDS), Parkinson's disease, cognitive dysfunction (including disorders of attention, focus and concentration), attention deficit syndrome, affective disorders, and for the control of pain. Thus modulation of the activity of acetylcholine receptors present on or within the cells of a patient suffering from any of the above-described indications will impart a therapeutic effect.

As employed herein, the phrase "an effective amount", when used in reference to compounds of the invention, refers to doses of compound sufficient to provide circulating concentrations high enough to impart a beneficial effect on the recipient thereof. Such levels typically fall in the range of about 0.001 up to 100 mg/kg/day; with levels in the range of about 0.05 up to 10 mg/kg/day being preferred.

The invention will now be described in greater detail with reference to the following non-limiting examples.

Example 1

Synthesis of Invention Ester Compounds Via Synthetic Scheme I

Formation of ester, Method A:

Into a three-neck, round-bottom flask fitted with an addition funnel, a condenser and flushed with argon was placed compound II, 2 mL/mmole of dry methylene chloride, triethylamine (1.1 eq.) and a catalytic amount of dimethylaminopyridine. To this mixture the acylchloride I (1.05 eq.) was added slowly at 0°C. The mixture was allowed to warm to room temperature and the reaction was monitored by thin layer chromatography (TLC). After completion, the reaction mixture was poured into water. The aqueous layer was basified with sodium carbonate and extracted three times with 3-4 mL/mmole of methylene chloride. The organic phases were combined, washed with 4-5 mL/mmole of brine, dried ($MgSO_4$) and concentrated under vacuum (15 mm Hg) to give an oil which was purified via chromatography on silica using a gradient of chloroform and methanol as eluant.

1-Methyl-2-pyrrolidinemethyl phenethylacetate (Method A):

(s)-(-)-1-Methyl-2-pyrrolidinemethanol (2.00 g, 17.4 mmole), triethylamine (1.93 g, 2.66 mL, 19.1 mmole), dimethylaminopyridine (0.01 g) and phenylacetyl chloride (2.81 g, 2.4 mL, 18.2 mmole) were stirred for 10 min at room temperature, yielding 1.01 g (4.32 mmole, 24%) of the desired compound. $^1H$ NMR (300 MHz, $CD_3Cl$) δ 7.28 (m, 5H), 4.07 (m, 2H), 3.65 (s, 1H), 3.02 (m, 1H), 2.45 (m, 1H), 2.35 (m, 3H), 2.29 (m, 1H), 1.85 (m, 1H), 1.7 (m, 2H), 1.55 (m, 2H); $^{13}C$ NMR (75.5 MHz, $CD_3Cl$) δ 171.5, 133.9, 129.2, 128.4, 126.9, 67.0, 63.6, 57.6, 41.3, 41.2, 28.2, 22.8.

1-Methyl-2-pyrrolidinemethyl 3-phenylpropionate (Method A):

(s)-(-)-1-Methyl-2-pyrrolidinemethanol (2.00 g, 17.4 mmole), triethylamine (1.93 g, 2.66 mL, 19.1 mmole), dimethyl-aminopyridine (0.01 g) and hydrocinnamoyl chloride (3.06 g, 2.7 mL, 18.2 mmole) were stirred for 10 min at room temperature, yielding 2.16 g (8.73 mmole, 50%) of the desired ester.

1-Methyl-2-pyrrolidinemethyl 3-phenylpropionate was converted to the fumarate salt (1.1 g, 3.03 mmole, 75%). $^1H$ NMR (300MHz, $CD_3OD$) δ 7.23 (m, 5H), 6.68 (s, 2H), 4.40 (m, 1H), 4.27(m, 1H), 3.60 (m, 2H), 3.11 (m, 1H), 2.92(m, 2H), 2.86 (s, 3H), 2.68 (m, 2H), 2.23(m, 1H), 2.05(m, 2H), 1.80 (m, 1H); $^{13}C$ NMR (75.5 MHz, $CD_3OD$) δ 174.1, 171.7, 142.2, 136.7, 130.0, 129.9, 127.8, 68.5, 63.2, 58.4, 41.3, 36.8, 32.1, 28.1, 23.5; mp 97-98°C; C H N Analysis $C_{15}H_{21}NO_2$ 1.0($C_4H_4O_4$).

Example 2

Synthesis of Invention Ester Compounds via Synthetic Scheme II

1-Methvl-2-pyrrolidinemethyl 3-(4-hydroxyphenyl)propionate

Into a two neck, round-bottom flask fitted with a condenser and flushed with nitrogen was placed (S)-(-)-1-methyl-2-pyrrolidinemethanol (10.4 g, 3.5 mmoles), 3-(4-tertbutyldimethylsillylhydroxyphenyl propionic acid N-hydroxysuccinimide ester (1.2 g, 3.18 mmole), a catalytic amount of *p*-toluenesulfonic acid (0.02 g) and anhydrous methylene chloride (10 mL). The mixture was refluxed for 12 hours, hydrolyzed with water (20 mL) and extracted with methylene chloride (3 x 25 mL). The organic layers were combined, washed with about 50 mL of brine, dried ($MgSO_4$) and concentrated under vacuum (15 mm Hg). The crude material was purified via chromatography on silica using chloroform as eluant yielding 1.13 g of pure material (3.01 mmole, 91%). The alcohol was deprotected using 1.1 eq of tetrabutylammonium fluoride in THF at room temperature for 30 min. After an aqueous work-up, the crude material was purified via chromatography on silica using chloroform/methanol (99:1) as eluant yielding 0.33 g of the desired ester (1.25 mmole, 95%).

$^1H$ NMR (300 MHz, $CDCl_3$) δ 6.92 (d, J=7Hz, 2H), 6.68 (d, J=7Hz, 2H) 4.12 (t, J=7Hz, 2H), 3.14 (m, 1H), 2.78 (m, 2H), 2.50 (m, 3H), 2.45 (s, 3H), 2.31 (m, 1H), 1.80 (m, 4H); $^{13}C$ NMR (75.5 MHz, $CD_3OD$) δ 173.3, 155.2, 131.3, 129.4, 129.1, 115.6, 115.5, 65.1, 64.2, 57.4, 41.3, 35.9, 29.9, 27.6, 22.3; mp 114-115°C; CHN Analysis: $C_{15}H_{21}NO_3$.

Example 3

Synthesis of Invention Thioether Compounds Via Synthetic Scheme IV

Formation of thioether (Method B):

Into a two neck flask fitted with a condenser, a thermometer and flushed with argon was placed 2-(2-chloroethyl)-1-methylpyrrolidine (1 eq), potassium carbonate (10 eq) and dry dimethylformamide (2 mL/mmole). The reaction mixture was heated at 70°C for 30 minutes, cooled to room temperature, and poured into 3 mL/mmole of a saturated solution of sodium bicarbonate (4 mL/mmole). The resulting mixture was extracted three times with 4 mL/mmole of ethyl acetate. The organic layers were combined, washed with brine, dried ($MgSO_4$) and concentrated under vacuum (0.01 mm Hg) to give an oil. The crude material was purified via chromatography on silica using a gradient of chloroform and methanol as eluant.

2-(2-Phenylthioethyl)-1-methylpyrrolidine (Method B):

2-(2-Chloroethyl)-l-methylpyrrolidine (1.30 g, 0.08 mmole), thiophenol (1.00 g, 9.08 mmole), potassium carbonate (12.56 g, 90.8 mmole) and DMF (18 mL) were combined, yielding 1.77 g (8.01 mmole, 89%) of the desired compound.

2-(2-Phenylthioethyl)-1-methylpyrrolidine (0.12 g, 0.54 mmole) was converted to the fumarate salt (0.17 g, 0.51 mmole, 94%). $^1H$ NMR (300 MHz, $CD_3OD$) δ 7.40 (m, 2H), 7.29 (m, 2H), 7.22 (m, 2H), 6.67 (s, 2H), 3.62 (m, 1H), 3.40 (m, 1H), 3.12 (m, 2H), 2.95 (m, 1H), 2.81 (s, 3H), 2.35 (m, 1H), 2.21 (m, 3H), 1.8 (m, 2H); $^{13}C$ NMR (75.5 MHz, $CD_3OD$) δ 171.5, 136.5, 136.3, 131.1, 130.3, 127.8, 68.8, 57.1, 39.5, 31.2, 30.8, 30.3, 22.4; mp 109-111°C; C H N Analysis $C_{13}H_{19}NS$ 1.0($C_4H_4O_4$).

2-[2-(4-Hydroxyphenyl)thioethyl]-1-methylpyrrolidine (Method B):

2-(2-Chloroethyl)-1-methylpyrrolidine (2.0 g, 13.5 mmole), 4-hydroxythiophenol (1.7 g, 13.5 mmole), potassium carbonate (18.7 g, 135.4 mmole) and DMF (26 mL) were combined, yielding 1.42 g (5.98 mmole, 44%) of the desired compound.

2-[2-(4-Hydroxyphenyl)thioethyl)-1-methylpyrrolidine (0.20 g, 0.84 mmole) was converted to the hydrochloride salt (0.13 g, 0.47 mmole, 56%). $^1$H NMR (300 MHz, $CD_3OD$) δ 7.22 (m, 2H), 6.67 (m, 2H), 3.52 (m, 1H), 3.35 (m, 1H), 3.05 (m, 1H), 2.86 (m, 1H), 2.76 (s, 3H) 2.68 (m, 1H), 2.26 (m, 1H), 1.95 (m, 3H), 1.64 (m, 2H); $^{13}$C NMR (75.5 MHz, $CD_3OD$) δ 159.3, 136.0, 124.8, 117.7, 69.5, 57.7, 40.2, 33.8, 31.6, 30.8, 22.9; mp 144-146°C; C H N analysis $C_{13}H_{19}NOS.HCl$.

2-[2-(3,4-Dichlorophenyl)thioethyl]-1-methylpyrrolidine (Method B):

2-(2-Chloroethyl)-1-methylpyrrolidine (1.50 g, 10.26 mmole), 3,4-dichlorobenzenethiol (1.82 g, 10.1 mmole), potassium carbonate (14.03 g, 101.6 mmole) and DMF (20 mL) were combined, yielding 0.46 g (4.34 mmole, 43%) of the desired compound.

2-[2-(3,4-Dichlorophenyl)thioethyl]-1-methylpyrrolidine (0.46 g, 1.58 mmole) was converted to the hydrochloride salt 0.36 g (1.10 mmole, 69%). $^1$H NMR (300 MHz, $CD_3OD$ δ 7.45 (m, 1H), 7.38 (m, 1H), 7.22 (m, 1H), 3.54 (m, 1H), 3.35 (m, 1H), 3.05 (m, 2H), 2.90 (m, 1H), 2.78 (s, 3H), 2.30 (m, 1H), 1.78-2.13 (m, 5H); $^{13}$C NMR (75.5 MHz, $CD_3OD$) δ 136.7, 132.9, 131.0, 130.6, 130.2, 128.9, 68.1, 56.3, 38.8, 29.9, 29.7, 29.4, 21.4; mp 115-116°C.

2-[2-(3-Fluoro-4-methylphenyl)thioethyl]-1-methylpyrrolidine (Method B) :

2-(2-Chloroethyl)-1-methylpyrrolidine (1.4 g, 9.48 mmole), 3-fluoro-4-methyoxythiophenol (1.5 g, 9.48 mmole), potassium carbonate (13.1 g, 94.8 mmole) and DMF (20 mL) were combined, yielding 1.90 g (7.03 mmole, 74%) of the desired compound.

2-[2-(3-Fluoro-4-methoxyphenyl)thioethyl]-1-methylpyrrolidine (0.905 g, 3.36 mmole) was converted to the fumarate salt 1.18 g (3.06 mmole, 91%). $^1$HNMR (300 MHz, $CD_3OD$) δ 7.12 (m, 2H), 6.95 (m, 1H), 6.57 (s, 2H), 3.55 (m, 1H), 3.32 (m, 1H), 2.97 (m, 2H) 2.78 (m, 1H), 2.73 (s, 3H), 2.23 (m, 1H), 1.97 (m, 3H), 1.88 (m, 2H); $^{13}$C NMR (75.5 MHz, $CD_3OD$) δ 171.11, 153.2 (d, J=20Hz) 148.5 (d, J=10Hz), 135.9, 129.0 (d, J=4Hz) 127.1 (d, J=6Hz) 120.0 (d, J=20Hz), 114.9 (d, J=2Hz), 68.45, 56.7, 56.5, 39.2, 32.36, 30.65, 30.0, 22.1; mp 104-105°C.

2-[2-(2-Chloro-4-hydroxyphenyl)thioethyl]-1-methylpyrrolidine (Method B):

2-(2-Chloroethyl)-1-methylpyrrolidine (2.85 g, 19.29 mmole), 2-chloro-4-hydroxythiophenol (3.1 g, 19.29 mmole), potassium carbonate (26.7 g, 192.9 mmole) and DMF (30 mL) were combined, yielding 3.2 g (11.77 mmole, 61%).

2-[2-(2-Chloro-4-hydroxyphenyl)-thioethyl]-1-methylpyrrolidine (0.575 g, 2.12 mmole) was converted to the fumarate salt (0.34 g, 0.78 mmole, 37%). $^1$HNMR (300 MHz, $CD_3OD$) δ 7.32 (d, J=7Hz, 1H), 6.82 (d, J=2Hz, 1H), 6.64 (dd, J=2Hz and 7 Hz, 1H), 6.59 (s, 1.4H), 3.55 (m, 1H), 3.38 (m, 1H), 3.0 (m, 2H), 2.73 (s, 3H) 2.7 (m, 1H), 2.28 (m, 1H), 1.98 (m, 3H), 1.75 (m, 1H); $^{13}$C NMR (75.5 MHz, $CD_3OD$) δ 173.3, 161.7, 140.9, 138.7, 138.1, 124,8, 119.9, 118.0, 70.6, 59.0, 41.4, 34.0, 32.7, 32.1, 24.2; mp 134-135°C; C H N analysis $C_{13}H_{18}ClNOS$ 1.4($C_4H_4O_4$).

Example 4

Radioligand Binding

$^3$H-Nicotine binding to rat cerebral membranes was performed according to modifications of the method of Flyn and Mash (*J. Neurochem.* **47**:1948 (1986)). $^3$H-Nicotine (80 ci/mmol; New England Nuclear Corporation, Boston, MA) was used as the ligand for nicotinic acetylcholine receptor binding assays. All other reagents were purchased from the Sigma Chemical Co. (St. Louis, MO).

Male Sprague-Dawley rats (250 - 400 gm) were sacrificed by decapitation, the brains removed and the cerebral cortex dissected on ice. Synaptic membranes were prepared by homogenizing the cortical tissue in 20 volumes of ice-cold modified Tris buffer (50 mM Tris pH 7.4, 120 mM NaCl, 5 mM KCl, 2 mM EDTA, 1 mM PMSF) with a polytron (20 sec at setting 5-6) followed by centrifugation (15 min at 25,000 x g) at 4°C. The resultant pellet was rehomogenized and centrifuged twice. The final pellet was resuspended in ice-cold assay buffer (50 mM Tris pH 7.4, 120 mM NaCl, 5 mM KCl, 2 mM $CaCl_2$, 1 mM $MgCl_2$) at a concentration of membrane equivalent to 1 gm wet weight cortex per 10 ml buffer. After protein determination the final membrane preparation was diluted with buffer to 3 mg protein/ml. This

membrane preparation was used in either the fresh state or frozen (-70°C) then thawed.

The binding assay is performed manually using 96-well plates, or using a Biomek automated work station (Beckman Instrument Co.). $^3$H-Nicotine was diluted in assay buffer to give a final concentration of 1.9 nM. The Biomek automated work station was programmed to automatically transfer 750 µl of assay buffer with $^3$H-nicotine, 230 µl of membrane preparation and 20 µl of solution containing the compound of interest in assay buffer, DMSO, ethanol:DMSO (1:1) or appropriate vehicle to the 96-well plate. Atropine was added to the incubation buffer at a final concentration of 3 µM to block binding to muscarinic acetylcholine receptor sites. The plates were maintained on ice for 60 min and the tissue-bound radioactivity was separated from the free by rapid filtration in a Brandel Harvester onto GF/C filters presoaked in 0.5% polyethyleneimine for at least 2 hr. The filters were washed with 4x2 ml of ice-cold assay buffer and filters were transferred to vials to which 4 ml of scintillation cocktail was added. The radioactivity was measured in a LS-6500 Beckman Liquid Scintillation Counter in an auto-dpm mode. Data were analyzed by log-logit transformation or non-linear regression analysis (e.g., employing GraphPad Prism, available from GraphPad Software, San Diego, CA) to give $IC_{50}$ values. Non-specific binding was defined by 10µM cytisine.

The ability of invention compounds to displace $^3$H-QNB (quinuclidinyl benzilate; 43 Ci/mmol) from muscarinic acetylcholine receptors in rat cerebral membranes was also tested using the above-described method in which $^3$H-nicotine was replaced with 60 pM $^3$H-QNB, and atropine was excluded from the incubation buffer.

The results of $^3$H-nicotine, $^3$H-cytisine and $^3$H-QNB binding/displacement assays of several invention compounds are summarized in Table I.

Table I

| Displacement of Radiolabeled Liqands | | | |
|---|---|---|---|
| | $IC_{50}$ (µM) | | |
| Compound tested, Formula I, wherein... | Nicotine | Cytisine | Quinuc Benz |
| A = 4-hydroxyphenyl;<br>B = absent;<br>D = -S-;<br>E = $-CH_2CH_2-$;<br>G = 1-methylpyrrolidine | 0.11 | 0.32 | 1.85 |
| A = 4-hydroxyphenyl;<br>B = $-CH_2CH_2-$;<br>D = -C(O)O-;<br>E = $-CH_2-$;<br>G = 1-methylpyrrolidine | 0.075 | 0.22 | >10 |
| A = 3-fluoro-4-methoxyphenyl;<br>B = absent;<br>D = -S-;<br>E = $-CH_2CH_2-$;<br>G = 1-methylpyrrolidine | 0.38 | 5.5 | 2.9 |
| A = 2-chloro-4-hydroxyphenyl;<br>B = absent;<br>D = -S-;<br>E = $-CH_2CH_2-$;<br>G = 1-methylpyrrolidine | 0.16 | 0.44 | 3.3 |
| A = phenyl;<br>B = $-CH_2CH_2-$;<br>D = -C(O)O-;<br>E = $-CH_2-$;<br>G = 1-methylpyrrolidine | 0.35 | 1.3 | 1.12 |

As evidenced by the $IC_{50}$ values in the Table, each of the compounds tested was able to displace acetylcholine receptor ligands from their binding sites in rat cerebral membranes.

Example 5

Neurotransmitter Release

Measurement of $^3$H-dopamine ($^3$H-DA) release from rat striatal slices was performed according to the method of Sacaan *et al*., (*J. Pharmacol. Comp. Ther* **224**:224-230 (1995)). Male Sprague-Dawley rats (250-300 g) were decapitated and the striata or olfactory tubercles dissected quickly on a cold glass surface. The tissue was chopped to a thickness of 300 μm with a McIlwain tissue chopper. After chopping again at right angles the tissue was dispersed and incubated for 10 min. at 37°C in oxygenated Kreb's buffer. $^3$H-Dopamine (40 Ci/mmol, NEN-Dupont, Boston, Ma) was added (50 nM) and the tissue was incubated for 30 min. in Kreb's buffer containing 10 μM pargyline and 0.5 mM ascorbic acid. Aliquots of the minced tissue were then transferred to chambers of a Brandel Superfusion system in which the tissue was supported on Whatman GF/B filter discs. The tissue was then superfused with buffer at a constant flow rate of 0.3 ml/min by means of a Brandel peristaltic pump. The perfusate was collected in plastic scintillation vials in 3-min fractions, and the radioactivity was estimated by scintillation spectrophotometry. The superfusate for the first 120 min was discarded. After two baseline fractions had been collected, the superfusion buffer was switched to fresh buffer with or without compound of interest. At the end of the experiment the filter and the tissue were removed, and the radiolabeled neurotransmitter content was estimated after extraction into scintillation fluid. The fractional efflux of radiolabeled neurotransmitter was estimated as the amount of radioactivity in the perfusate fraction relative to the total amount in the tissue.

Following essentially the same procedure as set forth in the preceding paragraph, the amount of $^3$H-norepinephrine $^3$H-NE) released from rat hippocampus, thalamus and prefrontal cortex slices superfused with buffer containing (or lacking) compounds of interest was also measured.

The results of studies of the effects of an invention compound (as compared to the effect of nicotine) on the release of neurotransmitters from rat brain slices are presented in Table II. The results presented in the Table are expressed as the percent fractional release.

Table II

| Ligand-Stimulated Neurotransmitter Release Data | | |
|---|---|---|
| | % of Nicotine Response[a] | |
| Ligand or Compound Tested, Formula I, wherein... | $^3$H-DA* Striatum | $^3$H-NE* Hippocampus |
| Nicotine | 100 (10μM) | 100 (300μM) |
| A = 4-hydroxyphenyl;<br>B = absent;<br>D = -S-;<br>E = $-CH_2CH_2-$;<br>G = N-methyl-2-pyrrolidine | 453 | 32 |
| A = 3-fluoro-4-methoxyphenyl;<br>B = absent;<br>D = -S-;<br>E = $-CH_2CH_2-$;<br>G = N-methyl-2-pyrrolidine | 21 | n.d. |
| A = 2-chloro-4-hydroxyphenyl;<br>B = absent;<br>D = -S-;<br>E = $-CH_2CH_2-$;<br>G = N-methyl-2-pyrrolidine | 800 | n.d. |
| A = 4-hydroxyphenyl;<br>B = absent;<br>D = -S-;<br>E = $-CH_2CH_2-$;<br>G = dimethylamino | 165 | n.d. |

* DA = Dopamine; NE = Norepinephrine
[a] Each compound was tested at 300 μM
[b] n.d. = not determined

Table II (continued)

| Ligand-Stimulated Neurotransmitter Release Data | | |
|---|---|---|
| | % of Nicotine Response[a] | |
| Ligand or Compound Tested, Formula I, wherein... | $^{3}$H-DA* Striatum | $^{3}$H-NE* Hippocampus |
| A = 4-hydroxyphenyl;<br>B = absent;<br>D = -S-;<br>E = $-CH_2-$;<br>G = N-methyl 7-azabicyclo[2.2.1]heptane | 660 | n.d. |

* DA = Dopamine; NE = Norepinephrine

[a] Each compound was tested at 300 µM

[b] n.d. = not determined

As shown in Table II, invention compound selectively induces release of catecholamines in different brain regions.

Example 6

Measurement of Acetylcholine Release in Rat Hippocampus

Male Sprague Dawley Rats (230-290 g) were anesthetized in a chamber saturated with isofluorane. Once immobilized, the rats were mounted in a Kopf stereotaxic apparatus with the incisor bar set at -3.3 mm (Praxinos and Watson, 1986). A midline incision was made on the skull to expose the underlying fascia. The skull was cleaned with an antibacterial preparation containing iodine and a hole was made in the skull at the following coordinates: AP, -3.5 mm and ML, +2.0 mm (Praxinos and Watson, 1986). A stainless steel guide cannula (Small Parts, Inc., Stillwater, FL) was cut to 2.0 mm length and inserted into the hole. Three additional holes were drilled into the skull surrounding the guide cannula and small machine screws were placed into these holes. The guide cannula and screws were secured by dental cement. A dummy cannula was inserted into the guide cannula to prevent clogging. The animals were removed from the stereotaxic frame and single housed for 3-7 days with free access to food and water.

On the day of the experiment, the rats were briefly anesthetized with isofluorane and the dummy cannulae were removed. A microdialysis cannula containing a Loop type probe with a rigid shaft and molecular weight cut-off of 6000 (ESA Inc, Chelmsfold, MA) of approximately 2 mm in length, was inserted into the guide cannula. Under these conditions, the microdialysis probe extended 2 mm beneath the guide cannula. The animal was placed in plastic bowl (CMA 120; CMA Microdialysis, Acton, MA, USA) with a harness around the neck. The microdialysis probe was connected to a syringe pump through which a salt solution representing the ionic concentration of the cerebrospinal fluid (artificial CSF; 145 mM NaCl; 27 mM KCl; 10 mM $MgCl_2$ and 12 mM $CaCl_2$; pH 7.4; Moghaddam and Bunney, J. Neurochem, 53, 652-654, 1989) containing 100 nM neostigmine was pumped at a rate of 1.5 µL/min.

Twenty minute fractions were collected and automatically injected via a sample loop and an auto-injector. The on-line microdialysis comprised of the following components: a CMA/100 microsyringe pump connected to a CMA/111 syringe selector. The mobile phase (100 mM disodium hydrogen phosphate; 2.0 mM 1-octane sulfonic acid sodium salt; 0.005% reagent MB (ESA Inc., Chelmeford, MA) pH 8.00 with phosphoric acid) was pumped using a model 580 ESA pump through a polymeric reverse phase column (ACH-3, ESA Inc., 3 µM spherical particles; 3.2 mmx 15 cm). The effluent from the column was passed through an enzyme reactor containing immobilized acetylcholinesterase and choline oxidase (ACH-SPR, ESA Inc.). The HPLC column and the enzyme reactor were placed in a housing with a constant temperature of 35°C. Acetylcholine and choline in microdialysis samples were converted into hydrogen peroxide which was detected by amperometric oxidation in a ESA model 5041 analytical cell containing a glassy carbon target electrode and a palladium reference electrode. The oxidation potential was 250 mV and the signal was detected by a ESA model 5200 A Coulochem detector. The retention times for choline and acetylcholine under these conditions were 4 and 6 min, respectively. The limit of detection for acetylcholine was less than 100 fmol on column.

On the day of the experiment, 10-12 fractions were collected to establish the baseline acetylcholine release. Following the establishment of baseline, rats were injected with the test compound and samples were collected until the levels of acetylcholine in the dialysate samples returned to baseline levels (3-5 hr). Compounds were typically dissolved in saline and the pH of the solution was adjusted by the addition of NaOH. An example of the results that may be obtained are demonstrated in the data below in which rats were injected with a compound according to Formula Z wherein A = 4-hydroxyphenyl, B is not present, D = -S-, E = $-CH_2CH_2-$, and G = 1-methylpyrrolidino, at a dose of 40 mg/kg in a volume of 0.2 cc/rat.

The effects of various treatments on hippocampal acetylcholine receptors are shown in Table 1 and Figures 1-5. The attenuation of acetylcholine release by mecamylamine induced by a compound having Formula Z wherein A = 4-hydroxyphenyl, B = not present, D = -S-, E = $-CH_2CH_2-$, and G = 1-methylpyrrolidino, arguing for the involvement of nicotinic acetylcholine receptors in this response.

Table III

| Treatment | Peak Increase, % of Baseline | Duration of Increase Above Baseline (min.) |
|---|---|---|
| Saline | 220 | 20 |
| Nicotine | 220 | 200 |
| Lobeline | 400 | 200 |
| Compound of Formula Z wherein:<br>A = 4-hydroxyphenyl,<br>B = not present,<br>D = -S-,<br>E = $-CH_2CH_2-$, and<br>G = N-methyl-2-pyrrolidino | 2,000 | 200 |
| Compound of Formula Z wherein:<br>A = 4-hydroxyphenyl,<br>B = not present,<br>D = -S-,<br>E = $-CH_2CH_2-$, and<br>G = $R^E$ and $R^F$ combine to form a 6-membered ring | 960 | 100 |
| Compound of Formula Z wherein:<br>A = 4-hydroxyphenyl,<br>B = $-CH_2-$,<br>D = -S-,<br>E = $-CH_2CH_2-$, and<br>G = N-methyl-2-pyrrolidino | 817 | 100 |

The effects of invention compounds on locomotor activity of rats were evaluated using the procedure of O'Neill *et al., Psychopharmacology* **104:**343-350 (1991). This assay can be used to assess the primary effect of a compound on general motor activity. A decrease in locomotor activity is indicative of a possible sedative effect on the animal, whereas an increase in locomotor activity is indicative of a stimulant effect on the animal.

Locomotor activity of rats (male Sprague-Dawley (Harlan) weighing 200-250 gm) was measured for 2 hrs in photocell cages immediately after administration of the invention compound. Prior to the test day, the animals were placed in the activity cages for 3 hrs to familiarize them with the experimental environment. On the test day, the animals were placed in the photocell cages and then injected with compound 1.5 hrs later.

The photocell cages were standard rodent cages (30 cm x 20 cm x 40 cm) with four infrared beams crossing the long axis. The animals were under no motivational constraints and were free to move around. Movements from one infrared beam to another (ambulation) were called "crossover"; successive interruptions of the same beam (vertical and other movements such as grooming) were called "general activity."

The results of one such study are shown in Table IV. Results are reported as the percent of change from control values (i.e., saline injection) for two post-injection periods: 0 - 60 minutes and 60 - 120 minutes, respectively.

## Claims

**1.** A compound having the Formula Z, as follows:

A-B-D-E-G (Z)

or enantiomers, diastereomeric isomers or mixtures of any two or more thereof, or pharmaceutically acceptable salts thereof

wherein:

A is

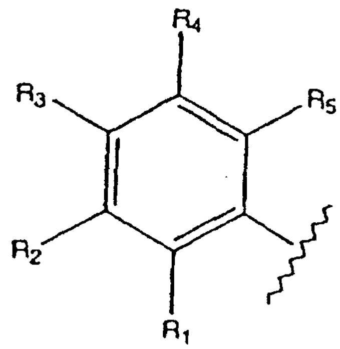

wherein:

each of $R_1$, $R_2$, $R_3$, $R_4$ and $R_5$ are independently selected from hydrogen, halogen, $C_{1-4}$ alkyl or $-OR_A$, wherein $R_A$ is selected from H, $C_{1-4}$ alkyl or $C_{6-14}$ aryl and wherein at least one of $R_1$, $R_2$, $R_3$, $R_4$ or $R_5$ is not hydrogen;

B is optionally present; with the proviso that when B is absent and D is -O-, at least one of $R_1$, $R_2$, $R_3$, $R_4$ or $R_5$ is not hydrogen, and when B is present, B is selected from $C_{1-4}$ alkylene, $C_{3-8}$ cycloalkylene, $C_{2-4}$ alkenylene or $C_{2-4}$ alkynylene;

D is selected from -O-, -C(O)-, -C(O)-O- or -S-;

E is selected from $C_{1-4}$ alkylene, $C_{2-4}$ alkenylene, or $C_{2-4}$ alkynylene, with the proviso that when any one of $R_1$, $R_2$, $R_3$, $R_4$ or $R_5$ is halogen, and D is -O-, then E is not methylene;

G is a dialkylamino group having the structure:

$$-N(R^E)\ (R^F),$$

wherein:

$R^E$ is hydrogen or a $C_{1-4}$ alkyl, and
$R^F$ is hydrogen or $C_{1-4}$ alkyl,

with the proviso that when G is dialkylamino, B is absent, D is -O-, and E is $C_{1-3}$ alkylene, then at least one of $R^1$ and $R^5$ is not $C_{1-4}$ alkyl, or

G is a nitrogen-containing cyclic moiety having the structure:

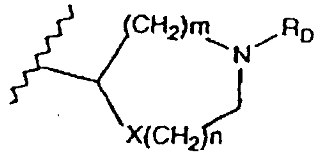

wherein:

m is 0-2
n is 0-3
X is optionally present, and when present is selected from -O-, $-CH_2O-$, -S-, $-CH_2S-$, -S(O)-, $-CH_2S(O)-$, $-S(O)_2-$, $-CH_2S(O)_2-$ or $-CH_2NH-$, wherein n is not 0 when X is not present, such that G is an aziridino, azetidino, tetrahydrooxazolo, tetrahydrothiazolo, pyrrolidino, piperidino, morpholino, thiomorpholino, piperazino, 7-azabicyclo[2.2.1]heptano, 8-azabicyclo[3.2.1]octano, 1-azabicyclo[2.2.2]octano or 9-azabicyclo[4.2.1]nonano, and

$R_D$ is selected from hydrogen, $C_{1-4}$ alkyl or $C_{3-4}$ cycloalkyl, or $R_D$ is absent when the nitrogen atom to which it is attached participates in the formation of a double bond, with the proviso that when D is -S-, then $R_D$ is not hydrogen or methyl,
with the proviso that:

when B is not present, D is -O- E is a $C_{1-3}$ alkylene, and G is a nitrogen-containing cyclic moiety wherein X is not present, then m and n combined ≠ 1; and
when B is not present, D is -S-, E is $-CH_2CH_2-$, G is a nitrogen-containing cyclic moiety wherein X is not present, m is zero, n is one and $R_D$ is $CH_3$, and each of $R_1$, $R_2$, $R_4$ and $R_5$ is H, then $R_3$ is not H, Cl or tert-butyl.

**2.** A compound according to claim 1 wherein G is an azetidino moiety or 1-methyl azetidino moiety.

**3.** A compound according to claim 1 wherein G is a piperidino moiety or 1-methylpiperidino moiety.

**4.** A compound according to claim 1, 2 or 3 wherein A is selected from 4-hydroxyphenyl, 2-chloro-4-hydroxyphenyl or 3-fluoro-4-methoxyphenyl.

**5.** A compound according to claim 1, 2, 3 or 4 wherein D is selected from -C(O)O- or -S-.

**6.** A compound according to claim 1 wherein G is pyrrolidino wherein X is not present, n = 2, m = 0 and $R_D$ is hydrogen or methyl.

**7.** A compound according to claim 1 wherein:

A = 2-chloro-4-hydroxyphenyl,
B = not present,
D = -S-,
E = $-CH_2CH_2-$, and
G = pyrrolidino wherein X is not present, n = 2, m = 0 and $R_D$ is hydrogen or methyl.

**8.** A compound according to claim 1 wherein:

A = 3-fluoro-4-methoxyphenyl,
B = not present,
D = -S-,
E = $-CH_2CH_2-$, and
G = pyrrolidino wherein X is not present, n = 2, m = o and $R_D$ is hydrogen or methyl.

**9.** A compound according to claim 1 wherein:

A = 4-hydroxyphenyl,
B = $-CH_2CH_2-$ or $-CH_2CH_2CH_2-$,
D = -C(O)O-,
E = $-CH_2-$, and
G = pyrrolidino wherein X is not present, n = 2, m = 0 and $R_D$ is hydrogen or methyl.

**10.** A compound according to claim 1 wherein:

A = 4-hydroxyphenyl,
B = $-CH_2CH_2-$,
D = -C(O)O-,
E = $-CH_2-$, and
G = pyrrolidino wherein X is not present, n = 2, m = 0 and $R_D$ is hydrogen or methyl.

**11.** A compound according to claim 1 wherein:

A = 4-hydroxyphenyl,
B = not present,
D = -S-,
E = $-CH_2CH_2-$, and
G = pyrrolidino wherein X is not present, n = 2, m = 0 and $R_o$ is hydrogen or methyl.

**12.** A compound according to claim 1 wherein:

A = 4-hydroxyphenyl,
B = $-CH_2-$,
D = -S-,
E = $-CH_2CH_2-$, and
G = pyrrolidino wherein X is not present, n = 2, m = 0 and $R_D$ is hydrogen or methyl.

**13.** A compound according to claim 1 wherein:

A = 2-fluoro-4-hydroxyphenyl,
B = not present,
D = -S-,
E = $-CH_2CH_2-$, and
G = pyrrolidino wherein X is not present, n = 2, m = 0 and $R_D$ is hydrogen or methyl.

**14.** A compound according to claim 1 wherein:

A = 2-methyl-4-hydroxyphenyl,
B = not present,
D = -S-,
E = $-CH_2CH_2-$, and
G = pyrrolidino, wherein X is not present, n = 2, m = 0 and $R_D$ is hydrogen or methyl.

**15.** A compound according to claim 1 wherein:

A = 4-hydroxyphenyl,
B = not present,
D = -O-,
E = $-CH_2-$, and
G = pyrrolidino, wherein X is not present, n = 2, m = 0 and $R_D$ is hydrogen or $C_1$-$C_4$ alkyl.

**16.** A compound according to claim 1 wherein:

A = 4-hydroxyphenyl,
B = $-CH_2-$,

D = -O-,
E = $-CH_2-$, and
G = pyrrolidino, wherein X is not present, n = 2, m = 0 and $R_D$ is hydrogen or $C_1$-$C_4$ alkyl.

**17.** A compound according to claim 1 wherein:

A = R-substituted phenyl, wherein R is defined the same as any one of $R^1$, $R^2$, $R^3$, $R^4$ or $R^5$,
B = not present,
D = -S-,
E = $-CH_2-$, and
G = pyrrolidino, wherein X is not present, n = 2, m = 0 and $R_D$ is hydrogen or $C_1$-$C_4$ alkyl.

**18.** A compound according to claim 1 wherein:

A = 4-hydroxyphenyl,
B = not present,
D = -S-,
E = $-CH_2-$, and
G = pyrrolidino, wherein X is not present, n = 2, m = 0 and $R_D$ is hydrogen or $C_1$-$C_4$ alkyl.

**19.** A compound according to claim 1 wherein:

A = 4-methoxyphenyl,
B = $-CH_2-$,
D = -S-,
E = $-CH_2-$, and
G = pyrrolidino, wherein X is not present, n = 2, m = 0 and $R_D$ is hydrogen or $C_1$-$C_4$ alkyl.

**20.** A compound according to claim 1 wherein:

A = 4-hydroxyphenyl,
B = $-CH_2-$,
D = -S-,
E = $-CH_2-$, and
G = pyrrolidino, wherein X is not present, n = 2, m = 0 and $R_D$ is hydrogen or $C_1$-$C_4$ alkyl.

**21.** A compound according to claim 1 wherein:

A = 4-hydroxyphenyl,
B = not present,
D = -S-,
E = $-CH_2-$, and
G = pyrrolidino, wherein X is not present, n = 2, m = 0 and $R_D$ is hydrogen or $C_1$-$C_4$ alkyl.

**22.** A compound according to claim 1 wherein:

A = 4-hydroxyphenyl,
B = not present,
D = -S-,
E = not present, and
G = 1-methyl-4-piperidino.

**23.** A compound according to claim 1 wherein:

A = 4-hydroxyphenyl,
B = not present,
D = -S-,
E = $-CH_2-$, and

G = 7-azabicyclo[2.2.1]heptane or N-methyl 7-azabicyclo[2.2.1]-heptane.

**24.** A compound according to claim 1 wherein:

A = 4-hydroxyphenyl,
B = not present,
D = -S-,
E = $-CH_2-$, and
G = 8-azabicyclo[3.2.1]octane or N-methyl 8-azabicyclo[3.2.1]-octane.

**25.** A compound according to claim 1 wherein;

A = 4-hydroxyphenyl,
B = not present,
D = -S-,
E = $-CH_2-$, and
G = 1-azabicyclo[2.2.2]octane or N-methyl 1-azabicyclo[2.2.2]-octane.

**26.** A compound according to claim 1 wherein:

A = 4-hydroxyphenyl,
B = not present,
D = -S-,
E = $-CH_2-$, and
G = 9-azabicyclo[4.2.1]nonane or N-methyl 9-azabicyclo[4.2.1]-nonane.

**27.** A compound according to claim 1 wherein:

A = 4-hydroxyphenyl,
B = not present,
D = -S-,
E = $-CH_2CH_2CH_2-$, and
G = dimethylamino.

**28.** A compound according to claim 1 wherein:

A = 4-hydroxyphenyl,
B = not present
D = -S-
E = $-(CH_2)_n-$, wherein n = 1-6, and
G = a dialkylamino group having the structure:

$$-N(R^E)\ (R^F),$$

wherein:

$R^E$ is hydrogen or a $C_{1-4}$ alkyl, and
$R^F$ is hydrogen or $C_{1-4}$ alkyl.

**29.** A compound as defined in any one of claims 1 to 28 for use in a method of modulating the activity of acetylcholine receptors, said method comprising:

contacting cell-associated receptors with a sufficient concentration of the compound to modulate the activity of said acetylcholine receptors.

**30.** A compound according to any one of claims 1 to 28 for use in the treatment of Parkinson's disease, Alzheimer's disease, dementia or pain.

**31.** A method for making compounds of structure Z according to claim 1, said method comprising contacting a compound of Formula II with a compound of Formula I' under ester-forming conditions, wherein compounds II and I', respectively, have the structures:

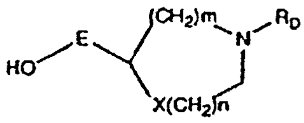

II

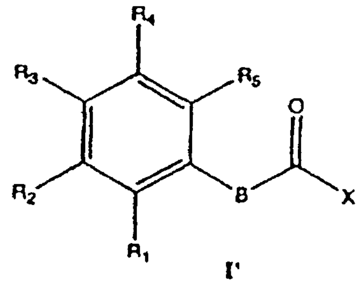

I'

wherein X = halogen, OH or OZ, wherein Z = alkyl or maleimido.

**Patentansprüche**

**1.** Eine Verbindung mit der folgenden Formel Z:

$$A\text{-}B\text{-}D\text{-}E\text{-}G \qquad (Z)$$

oder Enantiomere, diastereomere Isomere oder Mischungen aus beliebigen zwei oder mehreren davon oder pharmazeutisch annehmbare Salze davon,

wobei:

A

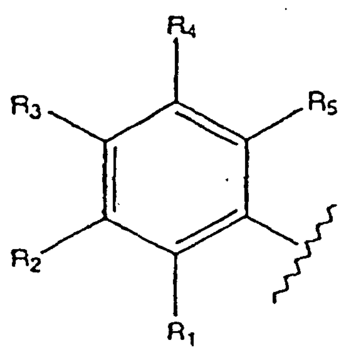

ist, wobei:

jeder der Reste $R_1$, $R_2$, $R_3$, $R_4$ und $R_5$ unabhängig ausgewählt ist aus Wasserstoff, Halogen, $C_{1-4}$-Alkyl oder -$OR_A$, wobei $R_A$ ausgewählt ist aus H, $C_{1-4}$-Alkyl oder $C_{6-14}$-Aryl und wobei wenigstens einer der Reste $R_1$, $R_2$, $R_3$, $R_4$ oder $R_5$ nicht Wasserstoff ist,

B gegebenenfalls vorhanden ist, mit der Maßgabe, daß, wenn B fehlt und D -O- ist, wenigstens einer der Reste $R_1$, $R_2$, $R_3$, $R_4$ oder $R_5$ nicht Wasserstoff ist, und daß, wenn B vorhanden ist, B ausgewählt ist aus $C_{1-4}$-Alkylen, $C_{3-8}$-Cycloalkylen, $C_{2-4}$-Alkenylen oder $C_{2-4}$-Alkinylen,

D ausgewählt ist aus -O-, -C(O) -C(O)- oder -S-,

E ausgewählt ist aus $C_{1-4}$-Alkylen, $C_{2-4}$-Alkenylen oder $C_{2-4}$-Alkinylen, mit der Maßgabe, daß, wenn irgendeiner der Reste $R_1$, $R_2$, $R_3$, $R_4$ oder $R_5$ Halogen ist und D -O- ist, E dann nicht Methylen ist,

G eine Dialkylaminogruppe mit der Struktur:

$$-N(R^E)(R^F) \text{ ist,}$$

wobei:

$R^E$ Wasserstoff oder ein $C_{1-4}$-Alkyl ist und
$R^F$ Wasserstoff oder $C_{1-4}$-Alkyl ist,

mit der Maßgabe, daß, wenn G Dialkylamino ist, B fehlt, D -Oist und E $C_{1-3}$-Alkylen ist, dann wenigstens einer der Reste $R^1$ und $R^5$ nicht $C_{1-4}$-Alkyl ist, oder

G ein stickstoffhaltiger cyclischer Rest ist mit der Struktur:

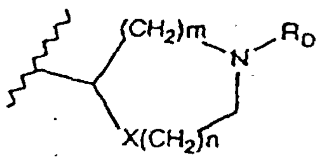

,

wobei:

m 0-2 ist,
n 0-3 ist,
X gegebenenfalls vorhanden ist, und wenn es vorhanden ist, ausgewählt ist aus -O-, $-CH_2O-$, -S-, $-CH_2S-$, -S(O)-, $-CH_2S(O)-$, $-S(O)_2-$, $-CH_2S(O)_2-$ oder $-CH_2NH-$, wobei n nicht 0 ist, wenn X nicht vorhanden ist, so daß G ein Aziridino, Azetidino, Tetrahydrooxazolo, Tetrahydrothiazolo, Pyrrolidino, Piperidino, Morpholino, Thiomorpholino, Piperazino, 7-Azabicyclo[2.2.1]heptano, 8-Azabicyclo[3.2.1]octano, 1-Azabicyclo [2.2.2]octano oder 9-Azabicyclo[4.2.1]nonano ist, und

$R_D$ ausgewählt ist aus Wasserstoff, $C_{1-4}$-Alkyl oder $C_{3-4}$-Cycloalkyl oder $R_D$ fehlt, wenn das Stickstoffatom, an das es gebunden ist, bei der Bildung einer Doppelbindung teilnimmt, mit der Maßgabe, daß, wenn D -S- ist, $R_D$ dann nicht Wasserstoff oder Methyl ist,
mit der Maßgabe, daß:

wenn B nicht vorhanden ist, D -O- ist, E ein $C_{1-3}$-Alkylen ist und G ein stickstoffhaltiger cyclischer Rest ist, wobei X nicht vorhanden ist, m und n dann zusammen $\neq$ 1 sind, und
wenn B nicht vorhanden ist, D -S- ist, E $-CH_2CH_2-$ ist, G ein stickstoffhaltiger cyclischer Rest ist, wobei X nicht vorhanden ist, m null ist, n eins ist und $R_D$ $CH_3$ ist und jeder der Reste $R_1$, $R_2$, $R_4$ und $R_5$ H ist, $R_3$ dann nicht H, Cl oder tert.-Butyl ist.

2. Eine Verbindung gemäß Anspruch 1, wobei G ein Azetidinorest oder ein 1-Methylazetidinorest ist.

3. Eine Verbindung gemäß Anspruch 1, wobei G ein Piperidinorest oder ein 1-Methylpiperidinorest ist.

4. Eine Verbindung gemäß Anspruch 1, 2 oder 3, wobei A ausgewählt ist aus 4-Hydroxyphenyl, 2-Chlor-4-hydroxyphenyl oder 3-Fluor-4-methoxyphenyl.

5. Eine Verbindung gemäß Anspruch 1, 2, 3 oder 4, wobei D ausgewählt ist aus -C(O)O- oder -S-.

6. Eine Verbindung gemäß Anspruch 1, wobei G Pyrrolidino ist, wobei X nicht vorhanden ist, n = 2, m = 0 und $R_D$ Wasserstoff oder Methyl ist.

**7.** Eine Verbindung gemäß Anspruch 1, wobei:

A = 2-Chlor-4-hydroxyphenyl,
B = nicht vorhanden,
D = -S-,
E = $-CH_2CH_2-$ und
G = Pyrrolidino, wobei X nicht vorhanden ist, n = 2, m = 0 und $R_D$ Wasserstoff oder Methyl ist.

**8.** Eine Verbindung gemäß Anspruch 1, wobei:

A = 3-Fluor-4-methoxyphenyl,
B = nicht vorhanden,
D = -S-,
E = $-CH_2CH_2-$ und
G = Pyrrolidino, wobei X nicht vorhanden ist, n = 2, m = 0 und $R_D$ Wasserstoff oder Methyl ist.

**9.** Eine Verbindung gemäß Anspruch 1, wobei:

A = 4-Hydroxyphenyl,
B = $-CH_2CH_2-$ oder $-CH_2CH_2CH_2-$,
D = -C(O)O-,
E = $-CH_2-$ und
G = Pyrrolidino, wobei X nicht vorhanden ist, n = 2, m = 0 und $R_D$ Wasserstoff oder Methyl ist.

**10.** Eine Verbindung gemäß Anspruch 1, wobei:

A = 4-Hydroxyphenyl,
B = $-CH_2CH_2-$,
D = -C(O)O-,
E = $-CH_2-$ und
G = Pyrrolidino, wobei X nicht vorhanden ist, n = 2, m = 0 und $R_D$ Wasserstoff oder Methyl ist.

**11.** Eine Verbindung gemäß Anspruch 1, wobei:

A = 4-Hydroxyphenyl,
B = nicht vorhanden,
D = -S-,
E = $-CH_2CH_2-$ und
G = Pyrrolidino, wobei X nicht vorhanden ist, n = 2, m = 0 und $R_D$ Wasserstoff oder Methyl ist.

**12.** Eine Verbindung gemäß Anspruch 1, wobei:

A = 4-Hydroxyphenyl,
B = $-CH_2-$,
D = -S-,
E = $-CH_2CH_2-$ und
G = Pyrrolidino, wobei X nicht vorhanden ist, n = 2, m = 0 und $R_D$ Wasserstoff oder Methyl ist.

**13.** Eine Verbindung gemäß Anspruch 1, wobei:

A = 2-Fluor-4-hydroxyphenyl,
B = nicht vorhanden,
D = -S-,
E = $-CH_2CH_2-$ und
G = Pyrrolidino, wobei X nicht vorhanden ist, n = 2, m = 0 und $R_D$ Wasserstoff oder Methyl ist.

**14.** Eine Verbindung gemäß Anspruch 1, wobei:

A = 2-Methyl-4-hydroxyphenyl,
B = nicht vorhanden,
D = -S-,
E = $-CH_2CH_2-$ und
G = Pyrrolidino, wobei X nicht vorhanden ist, n = 2, m = 0 und $R_D$ Wasserstoff oder Methyl ist.

**15.** Eine Verbindung gemäß Anspruch 1, wobei:

A = 4-Hydroxyphenyl,
B = nicht vorhanden,
D = -O-,
E = $-CH_2-$ und
G = Pyrrolidino, wobei X nicht vorhanden ist, n = 2, m = 0 und $R_D$ Wasserstoff oder $C_1$-$C_4$-Alkyl ist.

**16.** Eine Verbindung gemäß Anspruch 1, wobei:

A = 4-Hydroxyphenyl,
B = $-CH_2-$,
D = -O-,
E = $-CH_2-$ und
G = Pyrrolidino, wobei X nicht vorhanden ist, n = 2, m = 0 und $R_D$ Wasserstoff oder $C_1$-$C_4$-Alkyl ist.

**17.** Eine Verbindung gemäß Anspruch 1, wobei:

A = R-substituiertes Phenyl, wobei R genauso wie irgendeiner der Reste $R^1$, $R^2$, $R^3$, $R^4$ oder $R^5$ definiert ist,
B = nicht vorhanden,
D = -S-,
E = $-CH_2-$ und
G = Pyrrolidino, wobei X nicht vorhanden ist, n = 2, m = 0 und $R_D$ Wasserstoff oder $C_1$-$C_4$-Alkyl ist.

**18.** Eine Verbindung gemäß Anspruch 1, wobei:

A = 4-Hydroxyphenyl,
B = nicht vorhanden,
D = -S-,
E = $-CH_2-$ und
G = Pyrrolidino, wobei X nicht vorhanden ist, n = 2, m = 0 und $R_D$ Wasserstoff oder $C_1$-$C_4$-Alkyl ist.

**19.** Eine Verbindung gemäß Anspruch 1, wobei:

A = 4-Methoxyphenyl,
B = $-CH_2-$,
D = -S-,
E = $-CH_2-$ und
G = Pyrrolidino, wobei X nicht vorhanden ist, n = 2, m = 0 und $R_D$ Wasserstoff oder $C_1$-$C_4$-Alkyl ist.

**20.** Eine Verbindung gemäß Anspruch 1, wobei:

A = 4-Hydroxyphenyl,
B = $-CH_2-$,
D = -S-,
E = $-CH_2-$ und
G = Pyrrolidino, wobei X nicht vorhanden ist, n = 2, m = 0 und $R_D$ Wasserstoff oder $C_1$-$C_4$-Alkyl ist.

**21.** Eine Verbindung gemäß Anspruch 1, wobei:

A = 4-Hydroxyphenyl,
B = nicht vorhanden,

D = -S-,
E = $-CH_2-$ und
G = Pyrrolidino, wobei X nicht vorhanden ist, n = 2, m = 0 und $R_D$ Wasserstoff oder $C_1$-$C_4$-Alkyl ist.

**22.** Eine Verbindung gemäß Anspruch 1, wobei:

A = 4-Hydroxyphenyl,
B = nicht vorhanden,
D = -S-,
E = nicht vorhanden und
G = 1-Methyl-4-piperidino.

**23.** Eine Verbindung gemäß Anspruch 1, wobei:

A = 4-Hydroxyphenyl,
B = nicht vorhanden,
D = -S-,
E = $-CH_2-$ und
G = 7-Azabicyclo[2.2.1]heptan oder N-Methyl-7-azabicyclo[2.2.1]heptan.

**24.** Eine Verbindung gemäß Anspruch 1, wobei:

A = 4-Hydroxyphenyl,
B = nicht vorhanden,
D = -S-,
E = $-CH_2-$ und
G = 8-Azabicyclo[3.2.1]octan oder N-Methyl-8-azabicyclo[3.2.1]octan.

**25.** Eine Verbindung gemäß Anspruch 1, wobei:

A = 4-Hydroxyphenyl,
B = nicht vorhanden,
D = -S-,
E = $-CH_2-$ und
G = 1-Azabicyclo[2.2.2]octan oder N-Methyl-1-azabicyclo[2.2.2]octan.

**26.** Eine Verbindung gemäß Anspruch 1, wobei:

A = 4-Hydroxyphenyl,
B = nicht vorhanden,
D = -S-,
E = $-CH_2-$ und
G = 9-Azabicyclo[4.2.1]nonan oder N-Methyl-9-azabicyclo[4.2.1]nonan.

**27.** Eine Verbindung gemäß Anspruch 1, wobei:

A = 4-Hydroxyphenyl,
B = nicht vorhanden,
D = -S-,
E = $-CH_2CH_2CH_2-$ und
G = Dimethylamino.

**28.** Eine Verbindung gemäß Anspruch 1, wobei:

A = 4-Hydroxyphenyl,
B = nicht vorhanden,
D = -S-,
E = $-(CH_2)_n-$, wobei n = 1-6, und

G = eine Dialkylaminogruppe mit der Struktur:

$$-N(R^E)\ (R^F),$$

wobei:

$R^E$ Wasserstoff oder ein $C_{1-4}$-Alkyl ist und
$R^F$ Wasserstoff oder $C_{1-4}$-Alkyl ist.

**29.** Eine wie in irgendeinem der Ansprüche 1 bis 28 definierte Verbindung zur Verwendung bei einem Verfahren zur Modulierung der Aktivität von Acetylcholin-Rezeptoren, wobei das Verfahren umfaßt:

Inkontaktbringen von zellassoziierten Rezeptoren mit einer ausreichenden Konzentration der Verbindung, um die Aktivität der Acetylcholin-Rezeptoren zu modulieren.

**30.** Eine Verbindung gemäß irgendeinem der Ansprüche 1 bis 28 zur Verwendung bei der Behandlung von Parkinson-Krankheit, Alzheimer-Krankheit, Demenz oder Schmerz.

**31.** Ein Verfahren zur Herstellung von Verbindungen der Struktur Z gemäß Anspruch 1, wobei das Verfahren das Inkontaktbringen einer Verbindung der Formel II mit einer Verbindung der Formel I' unter Esterbildungsbedingungen umfaßt, wobei die Verbindungen II und I' die Strukturen:

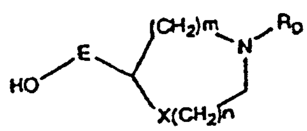

II

bzw.

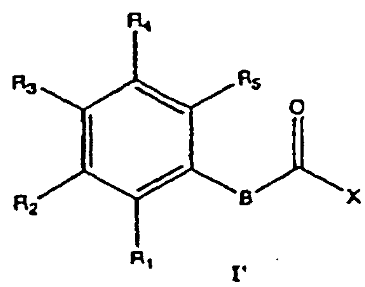

I'

besitzen, wobei X = Halogen, OH oder OZ, wobei Z = Alkyl oder Maleimido.

**Revendications**

**1.** Composé ayant la formule Z, comme suit :

$$A\text{-}B\text{-}D\text{-}E\text{-}G \qquad (Z)$$

ou des énantiomères, des isomères diastéréoisomères ou des mélanges de deux d'entre eux ou plus, ou des sels pharmaceutiquement acceptables de ceux-ci, où :

A est

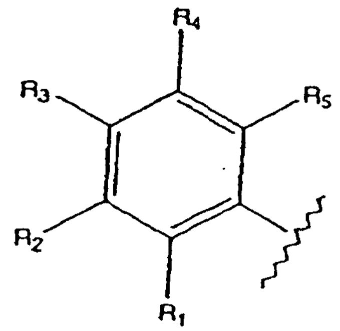

où chacun des $R_1$, $R_2$, $R_3$, $R_4$ et $R_5$ est, de façon indépendante, choisi parmi un hydrogène, un halogène, un reste alkyle en $C_{1-4}$ ou $-OR_A$, où $R_A$ est choisi parmi H, un reste alkyle en $C_{1-4}$ ou aryle en $C_{6-14}$ et où au moins un des $R_1$, $R_2$, $R_3$, $R_4$ ou $R_5$ n'est pas un hydrogène ;
B est présent de façon optionnelle ; sous réserve que si B est absent et D est -O-, au moins un des $R_1$, $R_2$, $R_3$, $R_4$ ou $R_5$ n'est pas un hydrogène et si B est présent, B est choisi parmi les restes alkylène en $C_{1-4}$, cycloalkylène en $C_{3-8}$, alcénylène en $C_{2-4}$ ou alcynylène en $C_{2-4}$;
D est choisi parmi -O-, -C(O)-, -C(O)-O- ou -S- ;
E est choisi parmi les restes alkylène en $C_{1-4}$, alcénylène en $C_{2-4}$ ou alcynylène en $C_{2-4}$, sous réserve que si l'un quelconque des $R_1$, $R_2$, $R_3$, $R_4$ ou $R_5$ est un halogène et D est -O-, alors E n'est pas un reste méthylène ;
G est un groupe dialkylamino ayant pour structure :

$$-N(R^E)(R^F),$$

où $R^E$ est un hydrogène ou un reste alkyle en $C_{1-4}$ et
$R^F$ est un hydrogène ou un reste alkyle en $C_{1-4}$,
sous réserve que, si G est un groupe dialkylamino, B est absent, D est -O- et E est un reste alkylène en $C_{1-3}$, alors au moins un parmi $R^1$ et $R^5$ n'est pas un reste alkyle en $C_{1-4}$ ou
G est un fragment cyclique contenant de l'azote ayant pour structure :

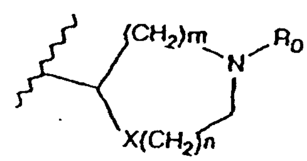

où

m est 0 à 2,
n est 0 à 3,
X est présent de façon optionnelle et, quand il est présent, il est choisi parmi -O-, $-CH_2O-$, -S-, $-CH_2S-$, -S(O)-, $-CH_2S(O)-$, $-S(O)_2-$, $-CH_2S(O)_2-$ ou $-CH_2NH-$, où n est différent de 0 si X n'est pas présent, si bien que G est un fragment aziridino, azétidino, tétrahydrooxazolo, tétrahydrothiazolo, pyrrolidino, pipéridino, morpholino, thiomorpholino, pipérazino, 7-azabicyclo[2.2.1]heptano, 8-azabicyclo[3.2.1]octano, 1-azabicyclo[2.2.2]-octano ou 9-azabicyclo[4.2.1]nonano et

$R_D$ est choisi parmi un hydrogène ou les restes alkyle en $C_{1-4}$ ou cycloalkyle en $C_{3-4}$, ou bien $R_D$ est absent si l'atome d'azote auquel il est attaché participe à la formation d'une double liaison, sous réserve que, si D est -S-, alors $R_D$ n'est pas un hydrogène ni un méthyle,

sous réserve que :

si B est absent, D est -O-, E est un reste alkylène en $C_{1-3}$ et G est un fragment cyclique contenant un azote

dans lequel X est absent, alors m et n combinés sont différents de 1 ; et
si B est absent, D est -S-, E est $-CH_2CH_2-$, G est un fragment cyclique contenant un azote dans lequel X est absent, m est zéro, n est un et $R_D$ est $CH_3$, et chacun des $R_1$, $R_2$, $R_4$ et $R_5$ est H, alors $R_3$ n'est pas H, Cl, ni tert-butyle.

**2.** Composé selon la revendication 1, dans lequel G est un fragment azétidino ou un fragment 1-méthylazétidino.

**3.** Composé selon la revendication 1, dans lequel G est un fragment pipéridino ou un fragment 1-méthylpiperidino.

**4.** Composé selon la revendication 1, 2 ou 3, dans lequel A est choisi parmi les restes 4-hydroxyphényle, 2-chloro-4-hydroxyphényle ou 3-fluoro-4-méthoxyphényle.

**5.** Composé selon la revendication 1, 2, 3 ou 4, dans lequel D est choisi parmi -C(O)-O- ou -S-.

**6.** Composé selon la revendication 1, dans lequel G est un fragment pyrrolidino dans lequel X est absent, n = 2, m = 0 et $R_D$ est un hydrogène ou un méthyle.

**7.** Composé selon la revendication 1, dans lequel :

A = 2-chloro-4-hydroxyphényle,
B = absent,
D = -S-,
E = $-CH_2CH_2-$, et
G = fragment pyrrolidino dans lequel X est absent, n = 2, m = 0 et $R_D$ est un hydrogène ou un méthyle.

**8.** Composé selon la revendication 1, dans lequel :

A = 3-fluoro-4-méthoxyphényle,
B = absent,
D = -S-,
E = $-CH_2CH_2-$, et
G = fragment pyrrolidino dans lequel X est absent, n = 2, m = 0 et $R_D$ est un hydrogène ou un méthyle.

**9.** Composé selon la revendication 1, dans lequel :

A = 4-hydroxyphényle,
B = $-CH_2CH_2-$ ou $-CH_2CH_2CH_2-$,
D = -C(O)O-,
E = $-CH_2-$, et
G = fragment pyrrolidino dans lequel X est absent, n = 2, m = 0 et $R_D$ est un hydrogène ou un méthyle.

**10.** Composé selon la revendication 1, dans lequel :

A = 4-hydroxyphényle,
B = $-CH_2CH_2-$,
D = -C(O)O-,
E = $-CH_2-$, et
G = fragment pyrrolidino dans lequel X est absent, n = 2, m = 0 et $R_D$ est un hydrogène ou un méthyle.

**11.** Composé selon la revendication 1, dans lequel :

A = 4-hydroxyphényle,
B = absent,
D = -S-,
E = $-CH_2CH_2-$, et
G = fragment pyrrolidino dans lequel X est absent, n = 2, m = 0 et $R_D$ est un hydrogène ou un méthyle.

**12.** Composé selon la revendication 1, dans lequel :

A = 4-hydroxyphényle,
B = $-CH_2-$,
D = -S-,
E = $-CH_2CH_2-$, et
G = fragment pyrrolidino dans lequel X est absent, n = 2, m = 0 et $R_D$ est un hydrogène ou un méthyle.

**13.** Composé selon la revendication 1, dans lequel :

A = 2-fluoro-4-hydroxyphényle,
B = absent,
D = -S-,
E = $-CH_2CH_2-$, et
G = fragment pyrrolidino dans lequel X est absent, n = 2, m = 0 et $R_D$ est un hydrogène ou un méthyle.

**14.** Composé selon la revendication 1, dans lequel :

A = 2-méthyl-4-hydroxyphényle,
B = absent,
D = -S-,
E = $-CH_2CH_2-$, et
G = fragment pyrrolidino dans lequel X est absent, n = 2, m = 0 et $R_D$ est un hydrogène ou un méthyle.

**15.** Composé selon la revendication 1, dans lequel :

A = 4-hydroxyphényle,
B = absent,
D = -O-,
E = $-CH_2-$, et
G = fragment pyrrolidino dans lequel X est absent, n = 2, m = 0 et $R_D$ est un hydrogène ou un reste alkyle en $C_{1-4}$.

**16.** Composé selon la revendication 1, dans lequel :

A = 4-hydroxyphényle,
B = $-CH_2-$,
D = -O-,
E = $-CH_2-$, et
G = fragment pyrrolidino dans lequel X est absent, n = 2, m = 0 et $R_D$ est un hydrogène ou un reste alkyle en $C_{1-4}$.

**17.** Composé selon la revendication 1, dans lequel :

A = un reste phényle substitué par R, où R a la même définition qu'un quelconque des $R_1$, $R_2$, $R_3$, $R_4$ ou $R_5$,
B = absent,
D = -S-,
E = $-CH_2-$, et
G = fragment pyrrolidino dans lequel X est absent, n = 2, m = 0 et $R_D$ est un hydrogène ou un reste alkyle en $C_{1-4}$.

**18.** Composé selon la revendication 1, dans lequel :

A = 4-hydroxyphényle,
B = absent,
D = -S-,
E = $-CH_2-$, et
G = fragment pyrrolidino dans lequel X est absent, n = 2, m = 0 et $R_D$ est un hydrogène ou un reste alkyle en $C_{1-4}$.

**19.** Composé selon la revendication 1, dans lequel :

A = 4-méthoxyphényle,
B = $-CH_2-$,
D = -S-,
E = $-CH_2-$, et
G = fragment pyrrolidino dans lequel X est absent, n = 2, m = 0 et $R_D$ est un hydrogène ou un reste alkyle en $C_{1-4}$.

**20.** Composé selon la revendication 1, dans lequel :

A = 4-hydroxyphényle,
B = $-CH_2-$,
D = -S-,
E = $-CH_2-$, et
G = fragment pyrrolidino dans lequel X est absent, n = 2, m = 0 et $R_D$ est un hydrogène ou un reste alkyle en $C_{1-4}$.

**21.** Composé selon la revendication 1, dans lequel :

A = 4-hydroxyphényle,
B = absent,
D = -S-,
E = $-CH_2-$, et
G = fragment pyrrolidino dans lequel X est absent, n = 2, m = 0 et $R_D$ est un hydrogène ou un reste alkyle en $C_{1-4}$.

**22.** Composé selon la revendication 1, dans lequel :

A = 4-hydroxyphényle,
B = absent,
D = -S-,
E = absent, et
G = 1-méthyl-4-pipéridino.

**23.** Composé selon la revendication 1, dans lequel :

A = 4-hydroxyphényle,
B = absent,
D = -S-,
E = $-CH_2-$, et
G = 7-azabicyclo[2.2.1]heptane ou N-méthyl-7-azabicyclo[2.2.1]heptane.

**24.** Composé selon la revendication 1, dans lequel :

A = 4-hydroxyphényle,
B = absent,
D = -S-,
E = $-CH_2-$, et
G = 8-azabicyclo[3.2.1]octane ou N-méthyl-8-azabicyclo[3.2.1]octane.

**25.** Composé selon la revendication 1, dans lequel :

A = 4-hydroxyphényle,
B = absent,
D = -S-,
E = $-CH_2-$, et
G = 1-azabicyclo[2.2.2]-octane ou N-méthyl-1-azabicyclo[2.2.2]-octane.

**26.** Composé selon la revendication 1, dans lequel :

A = 4-hydroxyphényle,
B = absent,
D = -S-,
E = $-CH_2-$, et
G = 9-azabicyclo[4.2.1]nonane ou N-méthyl-9-azabicyclo[4.2.1]nonane.

**27.** Composé selon la revendication 1, dans lequel :

A = 4-hydroxyphényle,
B = absent,
D = -S-,
E = $-CH_2CH_2CH_2-$, et
G = reste diméthylamino.

**28.** Composé selon la revendication 1, dans lequel :

A = 4-hydroxyphényle,
B = absent,
D = -S-,
E = $-(CH_2)_n-$, où n = 1 à 6, et
G = un groupe dialkylamino ayant pour structure :

$$-N(R^E)(R^F),$$

où $R^E$ est un hydrogène ou un reste alkyle en $C_{1-4}$ et
$R^F$ est un hydrogène ou un reste alkyle en $C_{1-4}$.

**29.** Composé tel que défini dans une quelconque des revendications 1 à 28 pour son utilisation dans une méthode de modulation de l'activité des récepteurs de l'acétylcholine, ladite méthode comprenant :

la mise en contact des récepteurs associés aux cellules avec une concentration du composé suffisante pour moduler l'activité desdits récepteurs de l'acétylcholine.

**30.** Composé selon l'une quelconque des revendications 1 à 28 pour son utilisation dans le traitement de la maladie de Parkinson, de la maladie d'Alzheimer, de la démence ou de la douleur.

**31.** Méthode de fabrication de composés de structure Z selon la revendication 1, ladite méthode comprenant la mise en contact d'un composé de formule II avec un composé de formule I' dans les conditions de formation d'un ester, les composés II et I', respectivement, ayant les structures :

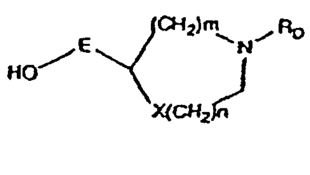

II

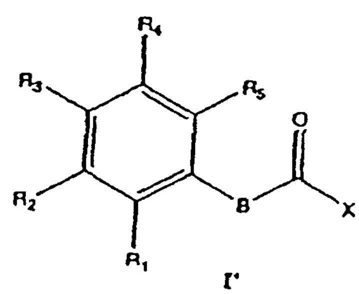

I'

où X = halogène, OH ou OZ, où Z = un reste alkyle ou maléimido.